(19) Europäisches Patentamt
European Patent Office
Office européen des brevets

(11) EP 4 245 777 A1

(12) EUROPEAN PATENT APPLICATION

(43) Date of publication:
**20.09.2023 Bulletin 2023/38**

(21) Application number: **22162499.2**

(22) Date of filing: **16.03.2022**

(51) International Patent Classification (IPC):
***C08F 2/38*** (2006.01) ***C08F 220/54*** (2006.01)
***A61K 9/50*** (2006.01) ***C08F 4/04*** (2006.01)
***C08F 8/30*** (2006.01)

(52) Cooperative Patent Classification (CPC):
(C-Sets available)
**C08F 2/38; A61K 9/5138; C08F 4/04; C08F 8/30; C08F 220/54;** C08F 2438/03 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Ludwig-Maximilians-Universität München**
**80539 München (DE)**

(72) Inventors:
- **MERKEL, Olivia**
  **82178 Puchheim (DE)**
- **ADAMS, Friederike**
  **70176 Stuttgart (DE)**
- **ZIMMERMANN, Christoph**
  **80799 München (DE)**
- **BALDASSI, Domizia**
  **80637 München (DE)**

(74) Representative: **Patent- und Rechtsanwälte Behrmann Wagner PartG mbB**
**Hegau-Tower**
**Maggistraße 5 (11. OG)**
**78224 Singen (DE)**

(54) **PROCESS FOR SYNTHESIZING POLY(SPERMINE ACRYLAMIDE) AND/OR POLY(SPERMINE ACRYLAMIDE-CO-N-ALKYLACRYLAMIDE) AND THEIR APPLICATION**

(57) The present invention relates to a process for the synthesis of poly(spermine acrylamide) and/or poly(spermine acrylamide-co-*N*-alkylacrylamide), the process comprising the following steps:
a) Reacting a mixture of *N*-acryloxysuccinimide with *N*-alkylacrylamide, and
b) Obtaining poly(*N*-acryloxysuccinimde) polymers, poly(*N*-alkylacrylamide) polymers and/or copolymers with varying ratios of N-acryloxysuccinimide and *N*-alkylacrylamide, and
c) Treating the obtained polymers and/or copolymers of step b) with at least 0.1 equivalent of tri-boc spermine per *N*-acryloxysuccinimiderepeating unit and obtaining poly(spermine acrylamide) and/or poly(spermine acrylamide-co-*N*-alkylacrylamide) polymers.

EP 4 245 777 A1

(52) Cooperative Patent Classification (CPC): (Cont.)

C-Sets
**C08F 220/54, C08F 220/34**

## Description

The invention refers to a process for the synthesis of poly(spermine acrylamide) and/or poly(spermine acrylamide-co-*N*-alkylacrylamide), a nanocarrier comprising them and their usage according to the genus of the independent claims.

RNA mediated targeted inactivation of genes, known as RNA interference (RNAi), is an established technique for the investigation of cellular processes in vivo and is increasingly being used for the potential treatment of a plurality of diseases. RNAi is a natural defense mechanism in which double-stranded RNA is fragmented into small RNA fragments, called small interfering RNA (siRNA) of 21 to 30 base pairs. The siRNA is then unwound and degrades the respective mRNA as a single-stranded guide siRNA, resulting in a reduced expression of the target gene. Since artificially synthesized siRNAs can be introduced into cells by transfection, in principle sequence-specific cleavage of any target mRNA (human, animal or foreign origin) can be achieved via a complementary siRNA. Since the discovery of RNAi, research interest in the field of RNA-based therapeutics has increased significantly. Despite intensive research, efficient intracellular delivery of siRNA across biological barriers, as well as a targeted delivery into specific cells turned out to be challenging, because the cells lack efficient nucleic acid uptake mechanisms. In addition, administration of "naked" and negatively charged siRNA is limited by rapid chemical degradation by proteins (e.g. serum nucleases) and immunological defense responses, especially when using intravenous administration. In addition, impermeability of siRNA through different barriers, e.g. air-blood-barrier, the lack of uptake mechanisms of cells for nucleic acids or high shear forces during nebulization still remain major problems.

It is therefore not surprising that many different viral vectors are known from the state of the art. Although viral vectors, e.g. lentiviruses, retroviruses or adeno-associated viruses, are the most efficient siRNA carriers, they are still associated with significant safety concerns due to their potential immunogenicity and mutagenicity. An alternative to these viruses are non-viral vectors, such as peptides, lipids or polymers. Especially cationic polymers are promising delivery systems due to their electrostatic interactions with siRNA resulting in polyplexes which protect the siRNA from external influences. These polycationic polymers can be synthesized on a large scale and generally exhibit higher biocompatibility than viral vectors. Polymers can be advantageous over lipids, since polyplexes can be converted into dry dosage forms by spray or freeze drying with significantly longer storage times. The development of polymers overcoming low transfection efficiencies of non-viral systems combined with minimal cytotoxicity remains to be the greatest barrier of successful non-viral nucleic acid delivery. In the past years, a multitude of different polymer ranging from biobased polymers such as chitosan and poly(L-lysine) to synthetic ones like polyethylenimine (PEI) and polymethacrylates were tested. Studies on structure-function relationships revealed a necessity of positively charged amines in which the structure and density of amines impacted the transfection efficiency. A higher density of amines and thus a higher charge provoke a higher efficiency, but also a higher toxicity. Amongst the polymers used for nucleic acid delivery, branched PEI showed one of the highest transfection efficiencies, making it one of the most widely used polymer as a non-viral vector. Its high buffering capacity is caused by its specific polymer architecture since it is a branched polymer consisting of primary, secondary and tertiary amino groups exhibiting pKa values distributed over the entire physiological pH range. However, as a consequence thereof, it also causes high cytotoxicity, which is associated with limitations and concerns in in vivo applications and with restrictions in clinical trials.

There is therefore a great need for the development of a non-viral, non-toxic, for the environment and the user harmless as well as highly reliable nanocarrier, which encapsulates and transports a substance in a targeted manner to an eukaryotic cell and which withstand several extracellular and/or intracellular barriers to release the substance at the site of action as an essential step for efficient therapy and/or treatment of a variety of diseases and/or disorders. Further, there is a need for said nanocarriers which can be generated and produced efficiently, on low cost and on large scale. The invention has therefore set itself the technical problem of providing a nanocarrier in order to overcome the above-mentioned difficulties and, above all, to ensure that a very high biocompatibility and non/very low cytotoxicity is exhibited in general.

This technical problem is solved in a surprisingly simple but effective manner by a process for synthesis of poly(spermine acrylamide) and/or poly(spermine acrylamide-co-*N*-alkylacrylamide), as well as a nanocarrier and a composition comprising these polymers and uses thereof according to the teachings of the independent claims.

According to the invention, a process for the synthesis of poly(spermine acrylamide) and/or poly(spermine acrylamide-co-*N*-alkylacrylamide) is suggested, the process comprising the following steps:

a) Providing a reaction mix, synthesizing and employing N-acryloxysuccinimide and linear or branched *N*-alkylacrylamide that are reacted via free radical polymerization using a polymerization initiator and/or synthesizing and employing *N*-acryloxysuccinimide and linear or branched *N*-alkylacrylamide that are reacted via reversible addition-fragmentation chain transfer polymerization using a chain-transfer agent and a polymerization initiator, and
b) Obtaining poly(*N*-acryloxysuccinimde) polymers, poly(*N*-alkylacrylamide) polymers and/or copolymers with varying ratios of *N*-acryloxysuccinimide and *N*-alkylacrylamide, and

c) Treating the obtained polymers and/or copolymers in step b) with at least 0.1 equivalent of tri-boc spermine per *N*-acryloxysuccinimide-repeating unit and obtaining poly(spermine acrylamide) and/or poly(spermine acrylamide-co-*N*-alkylacrylamide) polymers.

The method according to the invention is based on the basic idea that the endogenous molecule spermine has great potential in encapsulating a substance and, therefore, presents a promising non-viral delivery agent to a host organisms, in vivo and in vitro, like a cell, an organ and/or a tissue of a human and/or animal organism.

In the first step, a reaction mixture is provided and the precursor monomer *N*-acryloxysuccinimide 1 (NAS) is mixed with the hydrophobic unit of linear or branched *N*-alkylacrylamide, for example *N*-decylacrylamide 2 (DAA), having long or short alkyl chains. Linear *N*-alkylacrylamides A ($CH_2CHCONH(CH_2)nCH_3$) or branched *N*-alkylacrylamides B are used, having short or long alkyl chains (n = 1 - 20, m = 0-20, o = 0-20, p = 0-20 and m+o+p > 1). Examples of linear and branched *N*-alkylacrylamides are shown elsewhere herein. Monomer abbreviated with AlkAA and poly(*N*-alkylacrylamides) abbreviated with P(AlkAA).

N-acryloxysuccinimide 1 (NAS) is shown in formula (1):

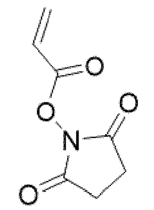

**1**

$C_7H_7NO_4$
169.14 g/mol

formula (1).

Linear *N*-alkylacrylamides A ($CH_2CHCONH(CH_2)_nCH_3$) are shown in formula (2) and branched or linear *N*-alkylacrylamides B, for example N-decylacrylamine, are shown in formula (3):

A

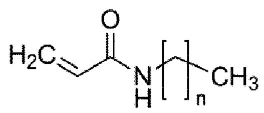

formula (2)

B

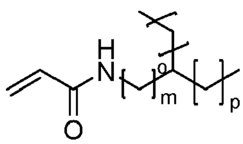

11

formula (3).

N-decylacrylamide 2 (DAA) is shown in formula (4):

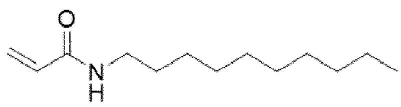

**2**

$C_{13}H_{25}NO$
211.35 g/mol

formula (4).

Preferably, the monomers *N*-acryloxysuccinimide 1 (NAS) and linear or branched *N*-alkylacrylamide (AlkAA), for example *N*-decylacrylamide 2 (DAA), were synthesized starting from acryloyl chloride, triethylamine and *N*-hydroxysuccinimide or *N*-decylamine, respectively, using known techniques. NAS polymerization was conducted for some time, preferable at least 30 min to 24 hours, more preferable at least 1 hour, 2 hours, 3 hours, 4 hours, 5 hours, 6 hours,7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours or 24 hours, at a temperature between 35°C to 90°C, more preferable at a temperature of at least 40°C, 45°C, 50°C, 55°C, 60°C, 65°C, 70°C, 75°C, 80°C, 85°C or 90°C, in a suitable and known solvent.

In the next step poly(*N*-acryloxysuccinimde) (P(NAS)) polymers, poly(*N*-alkylacrylamide (P(AlkAA)), for example poly(*N*-decylacrylamide) (P(DAA)), polymers and/or copolymers with varying ratios of NAS and AlkAA are obtained with yields of at least 60 %, preferable of at least 60 %, 61 %, 62 %, 63 %, 64 %, 65 %, 66 %, 67 %, 68 %, 69 %, 70 %, 71 %, 72 %, 73 %, 74 %, 75 %, 76 %, 77 %, 78 %, 79 %, 70 %, 81 % ,82 %, 83 %, 84 %, 85 %, 86 %, 87 %, 88 %, 89 %, 90 %, 91 %, 92 %, 93 %, 94 %, 95 %, 96 %, 97 %, 98 %, 99 %, 99,5 %, 99,6 %, 99,7 %, 99,8 %, 99,9 % or more. The term "obtaining" refers to yielding and/or isolating the polymers and/or copolymers using known techniques, like centrifugation, washing, precipitating and/or drying. Preferably, the obtained P(NAS) polymers, P(AlkAA) polymers and/or copolymers showed molar masses between 1 kg/mol and 80 kg/mol, more preferable at least 1 kg/mol, 2 kg/mol, 3 kg/mol, 4 kg/mol, 5 kg/mol, 6 kg/mol, 7 kg/mol, 8 kg/mol, 9 kg/mol, 10 kg/mol, 11 kg/mol, 12 kg/mol, 13 kg/mol, 14 kg/mol, 15 kg/mol, 16 kg/mol, 17 kg/mol, 18 kg/mol, 19 kg/mol, 20 kg/mol, 21 kg/mol, 22 kg/mol, 23 kg/mol, 24 kg/mol, 25 kg/mol, 26 kg/mol, 27 kg/mol, 28 kg/mol, 29 kg/mol, 30 kg/mol, 31 kg/mol, 32 kg/mol, 33 kg/mol, 34 kg/mol, 35 kg/mol, 36 kg/mol, 37 kg/mol, 38 kg/mol, 39 kg/mol, 40 kg/mol, 41 kg/mol, 42 kg/mol, 43 kg/mol, 44 kg/mol, 45 kg/mol, 46 kg/mol, 47 kg/mol, 48 kg/mol, 49 kg/mol, 50 kg/mol, 51 kg/mol, 52 kg/mol, 53 kg/mol, 54 kg/mol, 55 kg/mol, 56 kg/mol, 57 kg/mol, 58 kg/mol, 59 kg/mol, 60 kg/mol, 61 kg/mol, 62 kg/mol, 63 kg/mol, 64 kg/mol, 65 kg/mol, 66 kg/mol, 67 kg/mol, 68 kg/mol, 69 kg/mol, 70 kg/mol, 71 kg/mol, 72 kg/mol, 73 kg/mol, 74 kg/mol, 75 kg/mol, 76 kg/mol, 77 kg/mol, 78 kg/mol, 79 kg/mol or 80 kg/mol, and narrow to broad molecular weight distributions having a polydispersity D between 1.0 and 4.5 measured via known techniques, like size-exclusion chromatography (SEC) and/or spectroscopy, like $^{1}$H NMR spectroscopy and $^{13}$C NMR spectroscopy. Preferably, polymers and/or copolymers with varying ratios of NAS and AlkAA were synthesized in the first step by simple modulation of the monomer feed by preaddition mixing of different NAS/AlkAA ratios using the polymerization initiator in a range between 0,25 to 50 wt.%, more preferable at least 0,25 wt.%, 0,5 wt.%, 0,75 wt.%, 1 wt.%, 2 wt.%, 3 wt.%, 4 wt.%, 5 wt.%, 6 wt.%, 7 wt.%, 8 wt.%, 9 wt.%, 10 wt. %, 11 wt.%, 12 wt.%, 13 wt.%, 14 wt.%, 15 wt.%, 16 wt.%, 17 wt.%, 18 wt.%, 19 wt.%, 20 wt.%, 31 wt.%, 32 wt.%, 33 wt.%, 34 wt.%, 35 wt.%, 36 wt. %, 37 wt.%, 38 wt.%, 39 wt.%, 40 wt.%, 41 wt.%, 42 wt.%, 43 wt.%, 44 wt.%, 45 wt.%, 46 wt.%, 47 wt.%, 48 wt.%, 49 wt.% or 50 wt.%, and a total monomer concentration of 1 wt./vol.% to 90 wt./vol.%, more preferable at least 1 wt./vol.%, 2 wt./vol.%, 3 wt./vol.%, 4 wt./vol.%, 5 wt./vol.%, 6 wt./vol.%, 7 wt./vol.%, 8 wt./vol.%, 9 wt./vol.%, 10 wt./vol.%, 11 wt./vol.%, 12 wt./vol.%, 13 wt./vol.%, 14 wt./vol.%, 15 wt./vol.%, 16 wt./vol.%, 17 wt./vol.%, 18 wt./vol.%, 19 wt./vol.%, 20 wt./vol.%, 21 wt./vol.%, 22 wt./vol.%, 23 wt./vol.%, 24 wt./vol.%, 25 wt./vol.%, 26 wt./vol.%, 27 wt./vol.%, 28 wt./vol.%, 29 wt./vol.%, 30 wt./vol.%, 31 wt./vol.%, 32 wt./vol.%, 33 wt./vol.%, 34 wt./vol.%, 35 wt./vol.%, 36 wt./vol.%, 37 wt./vol.%, 38 wt./vol.%, 39 wt./vol.%, 40 wt./vol.%, 51 wt./vol.%, 52 wt./vol.%, 53 wt./vol.%, 54 wt./vol.%, 55 wt./vol.%, 56 wt./vol.%, 57 wt./vol.%, 58 wt./vol.%, 59 wt./vol.%, 60 wt./vol.%, 61 wt./vol.%, 62 wt./vol.%, 63 wt./vol.%, 64 wt./vol.%, 65 wt./vol.%, 66 wt./vol.%, 67 wt./vol.%, 68 wt./vol.%, 69 wt./vol.%, 70 wt./vol.%, 71 wt./vol.%, 72 wt./vol.%, 73 wt./vol.%, 74 wt./vol.%, 75 wt./vol.%, 76 wt./vol.%, 77 wt./vol.%, 78 wt./vol.%, 79 wt./vol.%, 80 wt./vol.%, 81 wt./vol.%, 82 wt./vol.%, 83 wt./vol.%, 84 wt./vol.%, 85 wt./vol.%, 86 wt./vol.%, 87 wt./vol. %, 88 wt./vol. %, 89 wt./vol. % or 90 wt./vol.%. Thus, it is known to the skilled person that when using fluid monomers solvents are not necessary. Thus, the ratios of NAS and AlkAA contents were obtained in the copolymers P(NAS-co-AlkAA) ranging from 99,9 mol% NAS to 0 mol% NAS. Further, the obtained homopolymers P(SpAA) and copolymers P(SpAA-co-AlkAA) showed different molecular weights and molar ratios of AlkAA ranging from 0 mol% to 99,9 mol%.

The terms "polymer" and "polymers" used herein are understandable to a person skilled in the art as interchangeable synonyms for each other to refer to a homopolymer or co-polymers, respectively.

In the next step, the obtained polymers and/or copolymers of the previous step were treated with at least 0.1 equivalent, preferably with varying amounts, more preferably with at least 0.2, 0.3, 0.4, 0.5, 0.6, 0.7, 0.8, 0.9, 1.0 or more equivalent, of tri-boc spermine 3 (TBSp) per NAS-repeating unit to obtain, preferably after purification using known techniques, tri-boc spermine acrylamide (TBSpAA), poly(spermine acrylamide) and/or poly(spermine acrylamide-co-*N*-alkylacrylamide) polymers. Thus, the active ester monomer was converted into spermine-based pendant groups by reaction with tri-boc spermine, optionally followed by deprotection described elsewhere herein. As shown elsewhere a set of three different homopolymers and/or three copolymers with varying molecular weights and ratios of hydrophobic AlkAA-units were obtained. Preferably, the tri-boc spermine has at least on free amine group, more preferably, tri-boc spermine is protected with one, two or three boc-protection groups and bears, respectively, three, two or one reactive and free amine group. Preferably, treatment, that means synthesis of protected spermine, is performed using known techniques, like orthogonal protection group chemistry in order to obtain boc-protected spermine.

The term "spermine" is known to the person skilled in the art and refers to a linear tetraamine consisting of two primary amines and two secondary amines having the formula $C_{10}H_{26}N_4$, that can be found in all eukaryotic cells. Tri-boc spermine 3 (TBSp) is shown in formula (5):

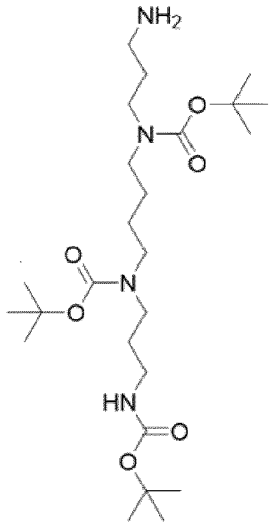

3

formula (5).

Preferably, the protonable unit of the homopolymers was calculated by dividing the mass of the repeating unit by the number of protonable primary and secondary amines present in the polymer. Structure and protonable unit of poly(spermine acrylamide) (P(SpAA) 1-3 are shown in formula (6):

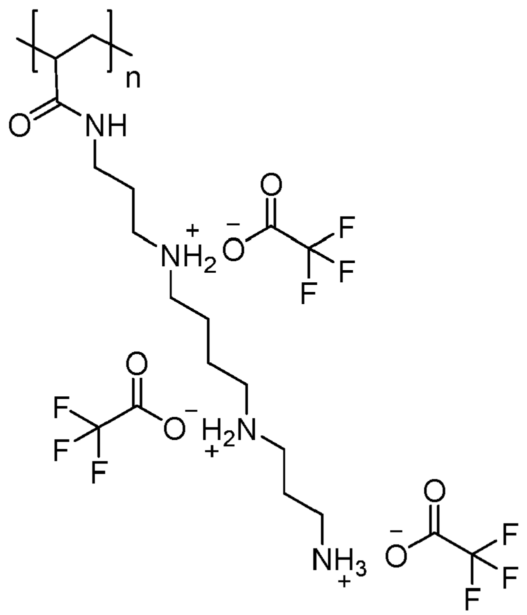

**P(SpAA)**

$M_n$ = 598.46 g/mol

$M_{protonable\ unit}$ = 199.49 g/mol

formula (6).

Preferably, the protonable unit of the different copolymers is calculated using the ratio of the two repeating

units and the number of protonable primary and secondary amines present in the polymer. Structure and protonable unit of poly(spermine acrylamide-co-DAA) (P(SpAA-co-DAA)) 1-3 are shown in formula (7):

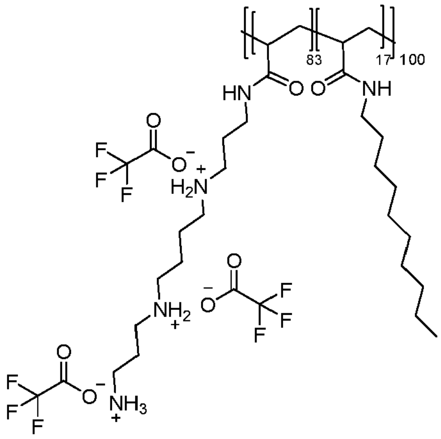

**P(SpAA-*co*-DAA)1**

$M_n$ = 53265.13 g/mol
$M_{protonable\ unit}$ = 213.9 g/mol

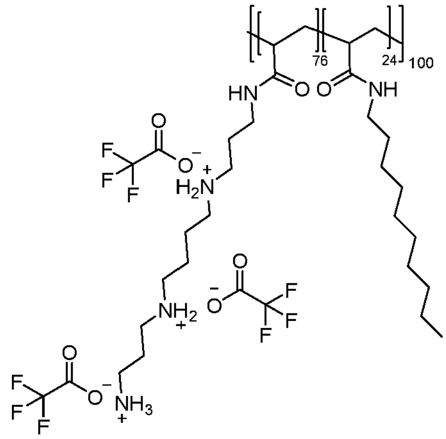

**P(SpAA-*co*-DAA)2**

$M_n$ = 50555.36 g/mol
$M_{protonable\ unit}$ = 221.7 g/mol

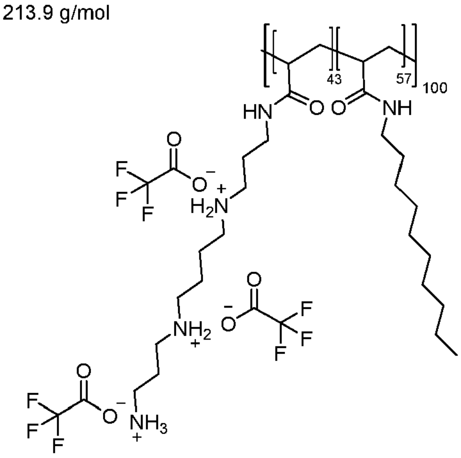

**P(SpAA-*co*-DAA)3**

$M_n$ = 37780.73 g/mol
$M_{protonable\ unit}$ = 292.9 g/mol

formula (7).

Thus, it has been found that said step c) is important for the synthesis of poly(spermine acrylamide) and/or poly(spermine acrylamide-co-*N*-alkylacrylamide) due to the fact that in said step possible unwanted side reactions are prevented and reaction of solely one amine group per spermine during post-polymerization functionalization are facilitated.

In addition, it has been found that modification with poly(ethylene glycol) can reduce aggregation or polyplex stabilization by hydrophilic shielding.

Thus, PEGylation of poly(spermine acrylamide) (P(SpAA)) and/or poly(spermine acrylamide-co-AlkAA) (P(SpAA-co-AlkAA)) polymers can be achieved by using PEG polymers as an end group or by using PEG-containing acrylamide monomers. Additionally, using AlkAA hydrophobic modifications with at least one or more alkyl chains can be performed which can have a beneficial effect on the cytotoxicity of polymers. Thus, it has been found that not only a reduction in molecular weight, but also modifications with hydrophilic, but uncharged, components further reduce the cytotoxicity of the polymers. Further additionally, it has been found that reactive groups at the end of the PEG chain, like azide groups, enable the click-chemistry with targeting molecules, for example folic acid, Transferrin, peptides, for targeted substance delivery to specific cells. Thus, using a PEG monomer or CTA together with co-*N*-alkylacrylamide, in particular N-alkylacrylamide, and NAS as monomers, triblock copolymers consisting of a hydrophilic, a hydrophobic

and a functional building block can be created via sequential addition of each monomer after the others are consumed or by isolating polymer blocks that are used as macroinitiators for polymerization of the next block. The order of the monomers can be interchanged. Further, it has been found that pre-mixing of monomers followed by copolymerization yields copolymers in which the copolymer structure is defined by the copolymerization parameter, i.e. the respective ratios of the reaction rate constants.

Surprisingly it has been found that poly(spermine acrylamide) polymers (P(SpAA)) and poly(spermine acrylamide-co-AlkAA) (P(SpAA-co-AlkAA)) copolymers have amphiphilic properties and having positively charged polymer groups which can electrostatically interact with negative charges of phosphate groups in of a substance. Thus, it has been found that post-polymerization functionalization with a modified spermine-species, in particular with optional deprotection, led to poly(spermine acrylamides) P(SpAA) with increased molecular-weights which still contain secondary and primary amines. Further surprisingly it has been found that the substance condensation ability and physicochemical characteristics, like particle size, size distribution and zeta potential, show that all synthesized polymers were able to form polyplexes with a substance at favorable sizes at around 100 nm in hydrodynamic diameter supplemented by narrow size distributions. These results in combination with slightly positive zeta-potentials and desired buffering-capacities indicate optimal conditions for efficient encapsulation. All homo- and copolymers showed an improved cellular uptake in cells compared to the commonly used PEI, like 25 kg/mol branched PEI. Moreover, P(SpAA-co-DAA)3, with a ratio of 43% cationic and 57% hydrophobic monomer subunits, was even able to induce a better cellular uptake than "gold standard" lipofectamine. Furthermore, using P(SpAA-co-DAA)3 similar or lower concentrations of most tested cytokines are shown in different, i.e. in vivo, studies elsewhere herein which qualifies these spermine based homo- and copolymers as promising agents for a variety of applications. Moreover, the process according to the invention can thus not only be carried out reliably and cost-efficiently, but also quickly and easily, especially since the few process steps can be carried out in a standardized manner with simple means and requires only equipment known to the skilled person.

Advantageous further embodiments of the invention, which can be implemented individually or in combination, are shown in the dependent claims.

In an embodiment of the present invention, it is conceivable that the reacting in step a) is via free radical polymerization (FRP) using a polymerization initiator and/or via reversible addition-fragmentation chain transfer (RAFT) polymerization by using a suitable chain-transfer agent (CTA), preferably 2-(dodecylthiocarbonothioylthio)-2-methylpropionic acid (CTA1) or 4-Cyano-4-[(dodecylsulfanylthiocarbonyl)sulfanyl]pentanoic acid (CTA2), and a polymerization initiator. Preferably, concentration ratios of the chain-transfer agent with respect to the polymerization initiator, whose concentration is specified elsewhere herein, range from 1/1 to 1/0.05. The reacting can occur in the presence of varying amounts of the polymerization initiator as the radical starter. As polymerization initiator any suitable and known compound can be used. Preferably, free radical polymerization of the active ester *N*-acryloxysuccinimide is performed. Preferably, the polymerizations are conducted in the presence of varying amount of monomer at fixed CTA concentrations. As chain transfer agents, all reagents suitable for promoting RAFT of acrylamides and acrylates can be used.

2-(dodecylthiocarbonothioylthio)-2-methylpropionic acid (CTA1) is shown in formula (8):

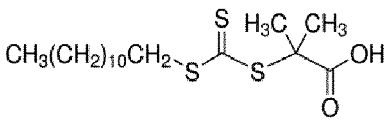

formula (8).

4-Cyano-4-[(dodecylsulfanylthiocarbonyl)sulfanyl]pentanoic acid (CTA2) is shown in formula (9):

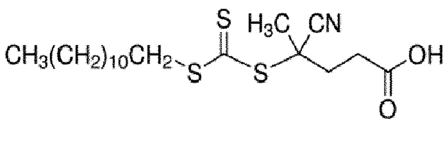

formula (9)

In an embodiment of the present invention, it is conceivable that in step c) at least one of the amine groups of the spermine reactant is protected using ethyl trifluoroacetate and/or di-tert-butyl dicarbonate. Thus, it has been found that said protection has the advantages of preventing possible unwanted side reactions and of facilitating reaction of solely one amine group per spermine during post-polymerization functionalization. Preferably, in the first step selectively protection of at least one, two or three of the primary amines with ethyl trifluoroacetate is performed at a temperature between -100°C to -50°C, preferably at least -50°C, -55°C, - 60°C, -65°C, -70°C, -75°C, -80°C, -85°C, -90°C, -95°C or -100°C, more preferable at -78°C. The remaining free amine groups are, in the next step, protected using di-tert-butyl dicarbonate ($Boc_2O$).

In an embodiment of the present invention, it is conceivable that in step c) a deprotection of the amine groups of tri-boc spermine acrylamide (TBSpAA), poly(spermine acrylamide) and/or poly(spermine acrylamide-co*N*-alkylacrylamide) polymers, preferably of all Boc-protected polymers, is performed resulting in poly(spermine acrylamide) and poly(spermine acrylamide-co-*N*-alkylacrylamide) polymers. Preferably, deprotection is performed using a diluted or concentrated acid well-known to the person skilled in the art in a concentration as required, as can be readily determined and/or ascertained by the skilled person. Thus, any suitable acid is used, preferably trifluoro-acetic acid (TFA), to obtain the TFA salts of the desired poly(spermine acrylamide) (P(SpAA)) and/or poly(spermine acrylamide-co-AlkAA) (P(SpAA-co-AlkAA)) polymers.

In an embodiment of the present invention, it is conceivable that in step c) ammonia is added. To ensure full removal of active esters to prevent side reactions during in vivo and/or in vitro evaluations, the reaction mixture was treated with ammonia to convert unreacted NAS units to water-soluble acrylamides.

In an embodiment of the present invention, it is conceivable that the polymerization initiator is azobisisobutyronitrile (AIBN) and/or a solvent is used wherein the solvent is dioxane, tetrahydrofuran, pyridine, dimethyl formamide, *N*-methyl-2-pyrrolidone and/or toluene, preferably using monomer concentrations specified elsewhere herein, further preferably in the presence of varying amounts of the polymerization initiator as the radical starter. Concentration of the polymerization initiator azobisisobutyronitrile (AIBN) has been described elsewhere herein.

It is understood that the definitions and expositions of the above terms apply *mutatis mutandis* to all aspects described below in this description, unless otherwise indicated.

According to the invention, further a nanocarrier comprising a non-viral delivery agent is suggested, wherein the non-viral delivery agent is poly(spermine acrylamide) and/or poly(spermine acrylamide-co-*N*-alkylacrylamide), preferably produced by a process described in detail elsewhere. The term "nanocarrier" is known to the person skilled in the art and refers to a carrier comprising a non-viral delivery agent being used as a transporter for any suitable substance or, preferably, comprising the non-viral delivery agent and the at least one substance.

In an embodiment of the present invention the nanocarrier comprises at least one substance, wherein the non-viral delivery agent is forming a complex, preferably a polyplex, with the substance. Thus, the non-viral delivery agent is a polymer, preferably P(SpAA)1, P(SpAA)3, P(SpAA-co-AlkAA), P(SPAA-co-DAA)1, P(SPAA-co-DAA)2 and/or P(SPAA-co-DAA)3, which is suitable for forming a complex, preferably a polyplex, with the substance. The terms "polyplex" and "polyplexes" are understandable to a person skilled in the art as interchangeable synonyms for each other to refer to a complex of a polymer, preferaby P(SpAA)1, P(SpAA)3, P(SpAA-co-AlkAA), P(SPAA-co-DAA)1, P(SPAA-co-DAA)2 and/or P(SPAA-co-DAA)3, with the substance. Surprisingly it has been found, that the nanocarrier has a buffering capacity of amine-containing polymers, which enables escape of the substance from the endosome before it is degraded by lysosomes. Thus, due to their amphiphilic properties poly(spermine acrylamide) polymer and/or poly(spermine acrylamide-co-*N*-alkylacrylamide) copolymer enhanced cellular uptake to a variety of different human and/or animal cells and enhanced stability of polyplexes, as well as a prevention of enzymatic degradation is achieved while the nanocarrier shows excellent substance encapsulation to obtain perfectly sized polyplexes at low N/P ratios. Thus, the nanocarrier protects the substance from degradation in the blood and prevents premature release of the substance from polyplexes caused by interaction or attachment of negatively charged serum proteins after administration of polyplexes. Moreover, it has been found that modification with poly(ethylene glycol) can reduce aggregation or polyplex destabilization by hydrophilic shielding.

Preferably, the polymers show full encapsulation at more than 0.3 μg, 0.35 μg, 0.4 μg, 0.45 μg, 0.5 μg, 0.55 μg, 0.6 μg, 0.65 μg, 0.7 μg, 0.75 μg, 0.8 μg, 0.85 μg, 0.9 μg, 0.95 μg, 1.0 μg, 1.05 μg, 1.1 μg, 1.15 μg or 1.2 μg polymer per μg substance. Preferably, the polyplex has a size between 40 and 180 nm, preferably at least 40 nm, 50 nm, 60 nm, 70 nm, 80 nm, 90 nm, 100 nm, 110 nm, 120 nm, 130 nm, 140 nm, 150 nm, 160 nm, 170 nm or 180 nm, and a positive zeta potentials of 1 - 35 mV, preferably of 3 - 23 mV. Further preferably, the N/P ratio is at least 1.0, 1.5, 2, 2.5, 3, 3.5, 4, 4.5, 5, 5.5, 6, 6.5, 7, 7.5, 8, 8.5, 9, 9.5, 10 or higher. The term "N/P ratio" is known to the person skilled in the art and defines the molar ratio between the polymers' amine groups (N) and the nucleic acids and/or amino acids phosphate groups (P). The amount of polymer needed to obtain different N/P ratios was calculated according to following equation (1):

$$m \text{ (polymer in pg)} = n \text{ substance (pmol)} \times \text{protonable unit (g/mol)} \times N/P \times \text{number of nucleotides substance.} \qquad \text{(eq. 1)}$$

In an embodiment of the present invention the substance is a nucleic acid, preferably a single or double stranded gene, DNA, cDNA, RNA, mRNA, RNAi, siRNA, shRNA, nucleotide, oligonucleotide or polynucleotide, and/or a nucleic acid dye. It will be understood by one skilled in the art that the terms "nucleic acid", "gene", "DNA", "cDNA", "RNA", "mRNA", "RNAi", "siRNA", "shRNA", "nucleotide", "oligonucleotide" and "polynucleotide" are used as interchangeable

synonyms for each other to refer to deoxyribonucleotides or ribonucleotides and polymers thereof, either single or double stranded. Thus, nucleic acids include, for example, but are by no means limited to, known nucleotide analogs or modified residues that are synthetic, naturally occurring and non-naturally occurring, that have similar binding properties to the nucleic acid and that are metabolized in a manner similar to the nucleic acid. For example, these include also phosphorothioates, phosphorami-dates, methyl phosphonates, chiral methyl phosphonates, 2-O-methyl ribonucleotides, peptide nucleic acids (PNAs). Further encompassed are also a conservatively modified variants thereof having complementary sequences, as well as having the explicitly stated sequence therof. In addition, degenerated codon substitutions can be obtained by generating sequences in which the third position of one or more selected (or all) codons is substituted with mixed bases and/or deoxyinosine residues. The term "nucleic acid dye" is known to the skilled person and refers to a colored substance that chemically binds to the nucleic acid to which it is being applied according to the manufacturer. The nucleic acid dye may have at least one feature, or all of the following features: relatively low "fluorescence background" (fluorescence in the absence of nucleic acids), if any, and ideally, no fluorescence background; relatively low toxicity, and ideally, no toxicity; relatively high fluorescent signal strength; and relative high stability. For example, the dye is an asymmetric cyanine dye, Nile red, a fluorogenic dye, ethidium bromide, propidium iodide, crystal violet/purple, 4',6-diamidino-2-phenylindole (DAPI), 7-aminoactinomycin D (7-AAD), PicoGreen, YOYO-1, Midori Green, SYBR Green I, SYBR Green II, SYBR Safe or SYBR Gold.

According to the invention, further a nanocarrier described in detail elsewhere and/or a pharmaceutical composition comprising a nanocarrier described in detail elsewhere and at least one further ingredient for use as a medicament for preventing the development, for slowing the progression and/or in the treatment of a disease and/or disorder is suggested, in particular a mental, neurodegenerative and/or neurological disease and/or disorder, a viral and/or bacterial infection, a respiratory or pulmonary disease, a metabolic disease, a metabolic syndrome and/or cancer.

The term "pharmaceutical composition" as used herein refers to a mixture of the nanocarrier as defined in detail elsewhere and at least one further ingredient. Preferably, such further ingredients may include stabilizing agents, wetting agents, pharmaceutical carriers, pharmaceutically acceptable carriers, diluents, pharmaceutically acceptable diluents, additional pharmaceutically active ingredients, release agents and the like. Preferred diluents include water, alcohols, physiological salt solutions, buffers such as phosphate buffered salt solutions, sugar solutions, syrup, oil, water, emulsions, various types of wetting agents and the like. The carrier must be acceptable in the sense that it is compatible with the other ingredients of the composition and that it is not harmful to the host organism. The pharmaceutical carrier used may comprise a solid, a gel or a liquid. Examples of solid carriers include lactose, terra alba, sucrose, talc, gelatin, agar, pectin, acacia, Magnesium stearate, stearic acid, and the like. Similarly, the carrier or diluent may include a time-release and/or time-delay material well known in the art, such as glycerol monostearate or glycerol indistearate alone or with a wax. Such suitable carriers include those mentioned above and others well known in the art, such as Remington's Pharmaceutical Sciences, Mack Publishing Company, Easton, Pennsylvania, European Pharmacopoeia, Homeopathic Pharmacopoeia of the United States, or HAB. The pharmaceutically acceptable diluent is selected so as not to interfere with the biological activity of the combination. Examples of such diluents include distilled water, physiological saline, Ringer's solutions, dextrose solution, and Hank's solution. In addition, the pharmaceutical composition may also contain other carriers, excipients, or non-toxic, non-therapeutic, non-immunogenic stabilizers and the like.

It is to be understood to the skilled person that the nanocarrier and/or the pharmaceutical composition are to be adapted for the appropriate use. Preferably, the nanocarrier and/or the pharmaceutical composition are formulated for dermal, intramuscular, subcutaneous, oral, rectal, retrograde, intravenous, local, intranasal, intratracheal, systemic, pulmonary and/or topical application, depending on the desired mode of administration. Preferably, oral application, for example in the form of tablets, solutions and/or drinking ampoules, or topical application in gel form or application by injection, for example as an active or passive vaccine, is provided. Further preferably, it was found that local administration routes can improve the performance of the in vivo substance, for example RNAi. Thus, intranasal or intratracheal delivery to airway and alveolar epithelium enhanced delivery efficiency of the substance, for example siRNA, regarding respiratory disorders by bypassing active targeting combined with a reduced dose compared to systemic administration. Thus, less systemic side effects and interactions with serum proteins are detected. For example, due to administration directly to nose or mouth, direct access to target lung epithelial cells is facilitated which are essential cell types in a variety of pulmonary diseases. The exact dosage recommended for each individual may depend, in principle, on other parameters well known to the skilled person. For example, children may receive a different dosage than adults. Whether the dosage needs to be adjusted can be readily determined by the skilled person using various known calculation tools.

The term "prevention the development of a disease and/or disorder" is also understandable to a person skilled in the art and relates to the partial, complete, short-term, permanent and/or long-term prevention of the onset of the respective disease and/or disorder as well as its prevention/prophylaxis. Preferably, this may be achieved by the nanocarrier which encapsulates and transports the desired substance in a targeted manner to the respective eukaryotic cell and which withstand several extracellular and/or intracellular barriers to release the substance at the site of action.

The term "slowing down the progression of a disease and/or disorder" is also understandable to a person skilled in the art and relates to the partial, complete, short-term, permanent and/or long-lasting maintenance of the achieved

condition, as well as an improvement of the symptoms associated therewith. Preferably, this may be achieved by the nanocarrier which encapsulates and transports the desired substance in a targeted manner to the respective eukaryotic cell and which withstand several extracellular and/or intracellular barriers to release the substance at the site of action.

The term "treatment" refers to any improvement of the human and/or animal organism that occurs in comparison to an untreated human and/or animal organism, preferably based on the release of the substance at the site of action in the human and/or animal organism as well as the prevention of the development and/or slowing of the progression of the disease and/or disorder. It is understood that treatment may not be successful in all of the human and/or animal organisms to be treated. However, the term implies that the treatment is successful for a statistically significant portion of the subjects (e.g., a cohort in a cohort study). Whether a portion is statistically significant can be readily determined by the skilled person using various known statistical evaluation tools, e.g., determination of confidence intervals, p-value determination, Student's t-test, Mann-Whitney test, etc. Preferred confidence intervals are at least 90%, at least 95%, at least 97%, at least 98%, or at least 99%. Preferred p-values are 0.05, 0.01, 0.005, or 0.0001.

The terms "medicament" and "drug" used herein are understandable to a person skilled in the art as interchangeable synonyms for each other to refer to a nanocarrier and/or a pharmaceutical composition in a therapeutically effective dose, as mentioned in detail elsewhere. Preferably, the pharmaceutical composition comprises the nanocarrier, at least one pharmaceutically acceptable carrier and/or a diluent. The pharmaceutical composition may be formulated for various routes of administration, as described in detail elsewhere. A therapeutically effective dose refers to the amounts necessary to prevent the development and/or slow the progression and/or in the treatment of the disease and/or disorder mentioned elsewhere. In this regard, therapeutic efficacy and toxicity can be determined by standard pharmaceutical procedures in cell cultures or experimental animals, e.g., ED50 (the dose that is therapeutically effective at 50%) and LD50 (the dose that is lethal at 50%). The dose ratio between therapeutic and toxic effects is the therapeutic index, which can be expressed as the LD50/ED50 ratio. The dosing regimen is determined by the treating physician and other clinical factors. As is well known in medicine, the appropriate dosage depends on many factors known to a professional, including size, body surface area, age, substance to be administered, sex, time and route of administration, general health status, and other concurrent medications. Progress can be monitored by periodic assessment.

The use of the nanocarrier and/or the pharmaceutical composition as a medicament may be in the treatment of diseases and/or disorders in the medical fields of oncology, infectology, dentistry, oral and maxillofacial medicine, oto-rhino-laryngology, ophthalmology, neurology, gynecology, gastroenterology, endocrinology, psychiatry, psychosomatics, orthopedics, pediatrics, surgery, urology, allergy and immunology, dermatology, internal medicine, obstetrics and gynecology, pediatrics, psychiatry, and/or the like. The terms "disease" and "disorder", as well as "morbidity", "sickness" and "illness" are understandable to a person skilled in the art as interchangeable synonyms for each other to refer, in medicine, to any condition, functional abnormality or disturbance that impairs the normal functioning of the human or animal body. It is, however, also known to the person skilled in the art that there are situations when specific terms are considered preferable. Preferably, possible diagnoses that may indicate the use of the nanocarrier and/or the pharmaceutical composition are, in addition to the aforementioned, among others, a mental, neurodegenerative and/or neurological disease and/or disorder, in particular an affective disorder, such as mania, hypomania, depression, a depressive disorder, bipolar disorder, a personality disorder, such as multiple, paranoid, schizoid, dissocial, emotionally unstable, histrionic, anancastic (obsessive-compulsive), anxious (avoidant), dependent (asthenic), and/or other personality disorder, a hallucination, an amnesia, a behavioral disorder, a drug and/or substance abuse, urticaria, a developmental disorder, a social disorder, an emotional disorder, a stress disorder, a dissociative disorder, a somatoform disorder, a neurorotic disorder, a body dysmorphic disorder, a phobia, a phobic disorder, an anxiety disorder, a panic disorder, an obsessive-compulsive disorder, schizophrenia, a schizotypal disorder, schizoaffective disorder, delusional disorder, psychosis, psychotic disorder, eating disorder, sleep disorder, sexual dysfunction, gender identity disorder, sexual preference disorder, postpartum mood crisis, tic disorder, Asperger's syndrome, attention deficit hyperactivity disorder, alcoholism, autism, Parkinson's disease, Alzheimer's disease, dementia, motor neuron disease, degenerative cerebellar disease, epilepsy, multiple sclerosis, irritable bowel syndrome, pain, like localized or whole body pain, such as headache and/or limb pain and/or migraine, a nerve injury, a tumor, localized, scattered or occurring throughout the body, preferably in the brain, spinal cord and/or peripheral nerves, a vascular disease, such as chemical cerebral infarction, cerebral hemorrhage, myocardial infarction, hypertension, circulatory disorder, Raynaud's disease and/or a functional localized vascular disorder, an inflammatory organic vascular disease, aneurysm, thrombosis, embolism and/or phlebitis, stroke, metabolic syndrome, diabetes mellitus type 1, diabetes mellitus type 2, gallbladder diseases, chronic diseases of the digestive tract, heart disease, chronic lower respiratory disease, cerebrovascular disease, chronic intestinal inflammations, elevated cholesterol levels, hyperacidity of the stomach, hypertension, dyslipidemia, respiratory problems, sleep apnea, coronary heart disease, osteoarthritis, gout, respiratory disorder or pulmonary disease, in particular severe acute respiratory syndrome virus, asthma, chronic obstructive pulmonary disease(COPD), chronic bronchitis, Emphysema, acute bronchitis and/or cystic fibrosis, rheumatic disease, brain disease, bacterial infection of the respiratory tract, the teeth, the mouth, the jaw, the eye, the musculoskeletal system, the blood, the gastrointestinal tract, preferably a bacterial infection of the colon, the small intestine, the duodenum, of the stomach, liver, gallbladder and/or

pancreas, skin, cardiovascular system, endocrine system, mind, immune system, nervous system, metabolism, wounds and/or genitourinary system, in particular strep throat, bacterial urinary tract infections (UTIs), bacterial food poisoning, bacterial cellulitis, such as due to Staphylococcus aureus (MRSA), bacterial vaginosis, gonorrhoea, chlamydia, syphilis, Clostridium difficile (C. diff), tuberculosis, whooping cough, pneumococcal pneumonia, bacterial meningitis, Lyme disease, cholera, botulism, tetanus and/or anthrax, viral infection, in particular severe acute respiratory syndrome coronavirus, influenza, common cold, measles, rubella, chickenpox, norovirus, polio, infectious mononucleosis, herpes simplex virus (HSV), human papillomavirus (HPV), human immunodeficiency virus (HIV), viral hepatitis A, B, C, D, and E, viral meningitis, West Nile Virus, rabies and/or ebola, cancer, in particular bladder, bone, brain, uterine, lung, melanoma, breast, cervical, pancreatic, hepatic, colon, esophageal, gastric, head & neck, mesothelioma, multiple myeloma, myelodysplastic syndrome, Non-Hodgkin's lymphoma, Hodgkin's lymphoma, leukemia, lymphatic, skin, blood, liver, ovarian, prostate, rectal, renal, sarcoma, testicular, thyroid and/or gallbladder cancer, sex hormone disorders, decreased libido, pain, increased risk of thrombosis and embolism and/or increased risk during surgery and anesthesia. Preferably, each is aimed at releasing the substance at the site of action by use of the nanocarrier as well as preventing and/or slowing down the development and/or progression of the respective disease and/or disorder, infection, syndrome and/or cancer as a major or minor outcome, thereby providing a good, preferably improved, prognosis for the treatment of the respective disease and/or disorder, infection, syndrome and/or cancer up to the cure thereof. A use of the nanocarrier and/or the pharmaceutical composition is thereby conceivable independently of the actual disease. A success is thereby seen in releasing the substance at the site of action and/or the prevention of the development and/or progression of the respective disease and/or disorder, infection, syndrome and/or cancer. It is known to a person skilled in the art that said disease and/or disorder may occur on its own or may be directly or indirectly related to an already known or not yet known above-mentioned disease and/or disorder.

According to the invention, further a nanocarrier described in detail elsewhere and/or a pharmaceutical composition comprising a nanocarrier described in detail elsewhere and at least one further ingredient for use in the treatment of a disease and/or disorder is suggested, in particular a mental, neurodegenerative and/or neurological disease and/or disorder, a viral and/or bacterial infection, a respiratory or pulmonary disease, a metabolic disease, a metabolic syndrome and/or cancer.

According to the invention, further a nanocarrier described in detail elsewhere and/or a pharmaceutical composition comprising a nanocarrier described in detail elsewhere and at least one further ingredient for use in a therapeutic procedure or in a non-therapeutic procedure for preventing the development, for slowing the progression and/or for the treatment of a disease and/or disorder is suggested, in particular a mental, neurodegenerative and/or neurological disease and/or disorder, a viral and/or bacterial infection, a respiratory or pulmonary disease, a metabolic disease, a metabolic syndrome and/or cancer.

Further details, features and advantages of the invention result from the following description of the preferred embodiments in connection with the dependent claims. Here, the respective features may be realized on their own or several in combination with each other. The invention is not limited to the preferred embodiments. The preferred embodiments are shown schematically in the figure. Identical reference numerals in the individual figures thereby designate identical or functionally identical elements or elements corresponding to one another with respect to their function.

In detail it is shown:

- **Fig. 1** schematic overviews over the synthesis of poly(spermine acrylamide) (P(SpAA)) and poly(spermine acrylamide-co-*N*-decylacrylamide) (P(SpAA-co-DAA), in particular via free-radical copolymerization (FRP; Fig. 1A), reversible addition-fragmentation chain transfer (RAFT) polymerization of homopolymers (Fig. 1B) and RAFT copolymerization of copolymers (Fig. 1C) and over the synthesis of P(SpAA-co-AlkAA) copolymers via FRP or RAFT (Fig. 1D), as well as over examples of linear *N*-Alkylacrylamides and branched N-Alkylacrylamides (Fig. 1E) and of polymers and copolymers accessible via free-radical (co)polymerization and/or via reversible addition-fragmentation chain transfer (RAFT) (co)polymerization (Fig. 1F); and
- **Fig. 2** a graph showing determination of copolymerization reactivity ratios; and
- **Fig. 3** graphs showing siRNA encapsulation profiles of polymers; and
- **Fig. 4** a graph showing siRNA encapsulation profiles of polymers; and
- **Fig. 5** graphs showing dynamic light scattering and laser Doppler anemometry measurements of polyplexes; and
- **Fig. 6** graphs showing release profiles of siRNA; and
- **Fig. 7** a graph showing cellular uptake of polyplexes; and
- **Fig. 8** a graph showing cellular uptake of polyplexes; and
- **Fig. 9** a graph showing enhanced green fluorescent protein (eGFP) knockdown; and
- **Fig. 10** an orthogonal view of phalloidin staining of Calu3 cells at ALI (left) and a graph showing GAPDH gene knockdown of P(SpAA-co-DAA)3 polyplexes (right); and
- **Fig. 11** a 3D view/orthoslice of phalloidin staining of Calu3 cells at ALI; and
- **Fig. 12** Thiazolyl blue tetrazolium bromide assays (Fig. 12A and 12B); and

**Fig. 13** graphs showing cytokine release in BALF after intratracheal instillation of siRNA polyplexes.

In **Fig. 1A,** a schematic overview over the synthesis of poly(spermine acrylamide) (P(SpAA)) and poly(spermine acrylamide-co-*N*-decylacrylamide) (P(SpAA-co-DAA) via free-radical (co)polymerization (FRP) of *N*-Acryloxysuccinimide (NAS) 1 and *N*-Decylacrylamide (DAA) 2, followed by post-polymerization with tri-boc spermine 3 and deprotection using trifluoroacetic acid (TFA) is shown. Further, in **Fig. 1B** and **Fig. 1C** schematic overviews over FRP as well as the reversible addition-fragmentation chain transfer (RAFT) (co)polymerization of NAS and DAA for homopolymers **(Fig. 1B)** and copolymers **(Fig. 1C)** are shown. Furthermore, in **Fig. 1D** a schematic overview over the synthesis of poly(spermine acrylamide-co-*N*-alkylacrylamide) (P(SpAA-co-AlkAA) via FRP as well as RAFT copolymerization of NAS and *N*-Alkylacrylamide (AlkAA) **(Fig. 1D)** is shown. In addition, schematic overviews over examples of linear *N*-Alkylacrylamides **A** ($CH_2CHCONH(CH_2)_nCH_3$) and branched N-Alkylacrylamides **B** with short or long alkyl chains (n = 1 - 20, m = 0-20, o = 0-20, p = 0-20 and m+o+p > 1) **(Fig. 1E)** and over examples of polymers and copolymers accessible via FRP and/or via RAFT (co)polymerization **(Fig. 1F)** are shown.

In **Fig. 2,** determination of copolymerization reactivity ratios according to Fineman-Ross technique is shown.

In **Fig. 3,** siRNA encapsulation profiles of polymers as measured by SYBR Gold assay at various polymer to siRNA weight ratios are shown. In **Fig. 4,** siRNA encapsulation profiles of polymers as measured by SYBR Gold assay at various N/P ratios are shown. In both Fig. 3 and Fig. 4, values are represented by the determined fluorescence of SYBR gold/siRNA complexes of uncondensed siRNA. In **Fig. 5,** dynamic light scattering and laser Doppler anemometry measurements of polyplexes formed with PEI, P(SpAA) 1-3 and P(SpAA-co-DAA) 1-3 are shown. In the top, hydrodynamic diameters (left y-axis), polydispersity indices (PDI, right y-axis) and (B) zeta potentials of polyplexes at N/P ratios of 2, 5, and 7. In the Fig. 3, Fig. 4 and Fig. 5 data points indicate mean $\pm$ SD, n = 3.

In **Fig. 6,** release profiles of siRNA from PEI, Spermine, P(SpAA) 1-3 and P(SpAA-co-DAA) 1-3 polyplexes at N/P 7 as a function of heparin concentration (0.0 - 1.0 I.U. heparin per well, 1 I.U. = 4.9 $\mu$g) at pH 7.4 (top) and pH 4.5 (bottom) are shown.

In **Fig. 7,** cellular uptake of polyplexes of PEI and P(SpAA) 1-3 at N/P ratios of 2,5 and 7 after 24 h of incubation, as quantified by flow cytometry performed with and without trypan quenching and presented as median fluorescence intensity are shown. In **Fig. 8,** cellular uptake of polyplexes of PEI at N/P 5 and P(SpAA-co-DAA) 1-3 at N/P ratios of 2.5 and 7 after 24 h of incubation, as quantified by flow cytometry performed with and without trypan quenching and presented as median fluorescence intensity are shown. Negative control in Fig. 7 and Fig. 8 with free siRNA treated cells. Positive control in Fig. 8 is Lipofectamin (LF).

In **Fig. 9,** enhanced green fluorescent protein (eGFP) knockdown of PEI, P(SpAA)2, P(SpAA-co-DAA)2 and P(SpAA-co-DAA)3 polyplexes in human non-small cell lung carcinoma cells expressing eGFP (H1299/eGFP) quantified by flow cytometry as median fluorescence intensity (MFI) of eGFP after transfection with polyplexes at N/P 2, 5 and 7 with eGFP siRNA (siGFP) or scrambled control siRNA (siNC) for 48 h without chloroquine treatment are shown. Blank samples consisted of H1299/eGFP untreated cells. The positive control consisted of Lipofectamin (LF) 2000 lipoplexes formulated with eGFP siRNA or scrambled control siRNA. Data given as MFI in %, normalized on each polyplex-siNC experiment.

In **Fig. 10, left** an orthogonal view of phalloidin staining of Calu3 cells at ALI is shown. Incubation time was 24 h; Blue: DAPI (nuclei); Green: Phalloidin (cytoskeleton); Red: AF647-siRNA. In **Fig. 10, right** GAPDH gene knockdown of P(SpAA-co-DAA)3 polyplexes using an N/P ratio of 5 in Calu3 cells grown in ALI cultures after 24 hr transfection. 100 pmol hGAPDH siRNA were used. Blank samples consisted of Calu3 monolayers treated with 5% glucose only. The positive control consisted of Lipofectamine 2000 (LF) lipoplexes with 100 pmol hGAPDH siRNA. GAPDH expression was normalized with $\beta$-actin expression and quantified by real time PCR. Data points indicate mean $\pm$ SD (n=3).

In **Fig. 11,** 3D view/orthoslice of phalloidin staining of Calu3 cells at ALI are shown. Incubation time was 24 h; Blue: DAPI (nuclei); Green: Phalloidin (cytoskeleton); Red: AF647-siRNA.

In Fig. 12, Thiazolyl blue tetrazolium bromide (MTT) assays to colorimetrically evaluate cell proliferation as well as the viability of the cells of all spermine-containing homo- and copolymers and PEI on the L929 mouse fibroblasts cell line after 24 h of incubation using different polymer concentrations are shown (Fig. 12A), as well as IC50 values calculated by plotting cell viability and concentration of polymers on a logarithmic scale using a sigmoidal model fit (Fig. 12B).

In **Fig. 13,** cytokine release in BALF after intratracheal instillation of siRNA polyplexes using PEI or P(SpAA-co-DAA)3 at an N/P ratio of 5. Data represent mean $\pm$ SEM (n= 4 for treatment with PEI or P(SPAA-co-DAA)3 polyplexes and n = 2 for untreated mice). Detection limit: dotted line.

The following preferred embodiments, shown in the examples below, serve only to illustrate the invention. They are not intended to limit the subject matter of the claims in any way.

## Examples:

### Example 1: (Co)polymerization of N-acryloxysuccinimide 1 (NAS) and N-decylacrylamide 2 (DAA)

Synthesis of 1-3

The monomers N-acryloxysuccinimide 1 (NAS) and N-decylacrylamide 2 (DAA) were synthesized starting from acryloyl chloride, triethylamine and N-hydroxysuccinimide or N-decylamine, respectively, using known techniques. Both monomers were isolated as pure products in good yields (71% NAS, 65% DAA). To use spermine as a functional molecule, tri-boc spermine 3 (TBSp) which is protected with three boc-protection groups and bears only one reactive primary amine was synthesized using orthogonal protection group chemistry in an adapted literature procedure. Such a complex synthesis route is mandatory to prevent possible unwanted side reactions and to facilitate reaction of solely one amine group per spermine during post-polymerization functionalization. In brief, the first step selectively protected one of the primary amines with ethyl trifluoroacetate at -78°C. The remaining free amine groups were protected using di-tert-butyl dicarbonate ($Boc_2O$). Increasing the pH of the reaction mixture to 11 led to selective deprotection of the acetate-protected amine followed by purification via column chromatography resulting in pure 3 as a colorless oil in moderate yield (48% yield).

Free radical polymerization of NAS and DAA; Polymerization studies

In order to increase the molecular weight of spermine, spermine homo- and copolymers were synthesized via free radical polymerization (FRP). Using NAS as a precursor monomer and DAA as the hydrophobic unit, these monomers were employed in FRP using azobisisobutyronitrile (AIBN) as polymerization initiator to evaluate the general activities, yields, molar masses, dispersities, and microstructures of the isolated poly(N-acryloxysuccinimide) (P(NAS)) and poly(N-decylacrylamide) (P(DAA)) polymers (see **Fig. 1A).** NAS polymerization was conducted over night at 65 °C in toluene using monomer concentrations of 10 wt./vol.% in the presence of varying amounts of AIBN (2-10 wt.%) as the radical starter. No clear correlation between weight ratio of AIBN and molecular weight during polymerization was observed (see Table 1). In Table 1 free radical polymerization of NAS and DAA and copolymerizations of NAS and DAA are shown, in toluene at 65 °C with monomer concentrations of 10 wt./vol.% over night. $M_n$ as obtained via SEC in DMF or chloroform relative to polystyrene standards. Đ = $M_w/M_n$ was calculated via SEC in DMF or chloroform. Ratio between NAS/DAA calculated via $^1H$ NMR spectroscopy in DMSO or $CDCl_3$. Ratio between TBSpAA/DAA calculated via $^1H$ NMR spectroscopy in $CDCl_3$. Ratio between SpAA/DAA calculated via $^1H$ NMR spectroscopy in $D_2O$. $M_n$ as obtained via SEC in chloroform relative to polystyrene standards. D = $M_w/M_n$ calculated via SEC in chloroform.

**Table 1: Free radical polymerization of NAS and DAA and copolymerizations of NAS and DAA**

| Entry | Name | NAS/ DAAFeed [mol%] | wt.% AIBN | Time [h] | Yield [%] | Mn [x103 g mol-1] | Đ | NAS/DAA[mol%] | Name Postpoly. | Mn [x103 g mol-1] | Đ | TBSpAA/ DAA[mol%] | Name Deprot. | SpAA/ DAA [mol%] |
|---|---|---|---|---|---|---|---|---|---|---|---|---|---|---|
| 1 | P(NAS)1-3 | 100/0 | 2 | 16.5 | 84 | 16.9 | 3.43 | 100/0 | P(TBSpAA)1-3 | 104.9 | 2.75 | 100/0 | P(SpAA)1-3 | 100/0 |
| 2 | | 100/0 | 5 | 17 | 97 | 20.0 | 3.46 | 100/0 | | 115.2 | 2.55 | 100/0 | | 100/0 |
| 3 | | 100/0 | 10 | 19 | 93 | 12.6 | 3.57 | 100/0 | | 97.8 | 2.11 | 100/0 | | 100/0 |
| 4 | P(DAA) | 0/100 | 2 | 16.5 | 80 | 23.7 | 2.02 | 0/100 | - | - | - | - | - | - |
| 5 | P(NAS-co-DAA)1-3 | 90/10 | 10 | 22 | 80 | 20.0 | 3.40 | 91/9 | P(TBSpAA-co-DAA)1-3 | 53.1 | 3.5 | 83/17 | P(SpAA-co-DAA)1-3 | 83/17 |
| 6 | | 80/20 | 10 | 21 | 72 | 29.3 | 2.8 | 83/17 | | 82.5 | 2.61 | 72/28 | | 76/24 |
| 7 | | 50/50 | 10 | 20 | 87 | 35.3 | 3.69 | 49/51 | | 129.5 | 1.74 | 40/60 | | 43/57 |

As expected for FRP and due to precipitation of the polymer during polymerization, the obtained P(NAS) polymers 1-3 showed molar masses between 12.6 kg/mol and 20.0 kg/mol and broad molecular weight distributions ($3.43 \leq Đ \leq 3.57$) measured via size-exclusion chromatography (SEC) in dimethylformamide (DMF) relative to polystyrene standards. Polymers were isolated via centrifugation, washing with toluene, followed by drying overnight. All polymers were isolated in good yields (84 - 97%).

A similar procedure was used for DAA polymerization. Using 2 wt.% AIBN, P(DAA) with a molar mass of 23.7 kg/mol and a polydispersity of 2.02 was isolated after precipitation from acetonitrile. Due to insolubility of P(DAA) in DMF, SEC was measured in chloroform relative to polystyrene standards. Additionally, copolymers with varying ratios of NAS and DAA were synthesized by simple modulation of the monomer feed by preaddition mixing of different NAS/DAA ratios using 10 wt.% AIBN and a total monomer concentration of 10 wt./vol. %. Different ratios of NAS and DAA contents were obtained in the copolymers P(NAS-co-DAA) 1-3 ranging from 90 mol% NAS to 50 mol% NAS (see Table 1). The polymers were obtained with yields between 72 and 87%. $^1$H NMR spectroscopy verified the successful polymerization since all signals of the NAS and DAA repeating units agree with those of the obtained homopolymers P(NAS) and P(DAA). Composition of NAS/DAA was calculated via $^1$H NMR spectroscopy of the dried polymer using the methyl group of DAA (0.8 ppm) and the signal at 2.8 ppm consisting of 4 protons of NAS.

Whereas P(NAS-co-DAA) 1 and 2 were still soluble in deuterated DMSO due to high ratios of NAS, NMR analysis of P(NAS-co-DAA)3 with 50mol% DAA was performed in $CDCl_3$. It was possible to exactly tune the composition of the copolymer through the monomer feed as the ratios of the two monomers in the feed and in the polymer were nearly identical (Table 1). DOSY NMR studies were used to confirm the successful linkage between the NAS and the DAA monomers and to exclude the formation of two separate homopolymers. DOSY NMR spectra of P(NAS-co-DAA)2 showed only one set of signals assigned to the diffusion coefficient for the copolymer. Molar masses and polydispersities were calculated via SEC in DMF relative to polystyrene standards. Compared to homopolymerizations, the shift in

molecular weight of the copolymers with increasing DAA content can either be attributed to higher molecular weights or to different interactions of DAA containing polymers with the SEC-column.

Free radical polymerization of NAS and DAA; Copolymerization Parameter To further understand the chemical composition of the copolymers, a series of reactions with different monomer feed ratios was performed to determine the monomer reactivity ratios of NAS and DAA in copolymerizations. Fineman-Ross technique led to a term which gives -after linear fit - (F/f)(f - 1) as ordinate and ($F^2$/f) as abscissa resulting in $r_1$($r_{NAS}$) as the slope (see **Fig. 2;** left). Rearranging this term to another equation, (f-1)/F can be plotted against (f/$F^2$) resulting in minus $r_2$ ($r_{DAA}$) as the slope (see **Fig. 2;** right). $r_1$ ($r_{NAS}$) was calculated as 0.76, whereas $r_2$ ($r_{DAA}$) had a value of 1.39. The reactivity ratio of NAS is between 0 and 1 indicating that the growing radical chain ending with a NAS unit preferred a DAA monomer over NAS monomer during propagation. In accordance, also DAA-radical chain ends preferred reaction with a DAA monomer ($r_{DAA}$ is slightly over 1), but does not only allow homopolymerization, resulting in DAA-blocks interrupted by NAS-monomers. Such a block structure would be beneficial for micellization approaches due to separation of DAA and NAS units leading to block structures of DAA until it is consumed followed by regions where NAS fractions are higher.

Reversible addition-fragmentation chain transfer of NAS and DAA; Polymerization studies

In addition to spermine homo- and copolymers synthesized via free radical polymerization, spermine homo- and copolymers were also synthesized via RAFT polymerization using 2-(dodecylthiocarbonothioylthio)-2-methylpropionic acid (CTA1) or 4-Cyano-4-[(dodecylsulfanylthiocarbonyl)sulfanyl]pentanoic acid (CTA2) as the CTA (see **Fig. 1B** and **Fig. 1C).** The polymerizations were conducted in the presence of varying amount of monomer at fixed CTA concentrations. An advantage of RAFT polymerization is that the absolute molar masses can be directly calculated via $^1$H NMR spectroscopy by comparing distinctive signals of the pendant groups and the end group. Further, the living character of RAFT allows lower molecular weight distributions and the synthesis of block copolymers. In Table 2 polymerization of NAS using a CTA1/RAFT is shown, in Table 3 polymerization of NAS using a CTA2/RAFT is shown and in Table 4 Polymerization of NAS and DAA using CTA1 is shown. In Tables 2 and 3 2 mg/mL solution of AIBN in the used solvent was used, whereas in Table 4 either a 2 mg/mL solution of AIBN in DMF or 2 M solution of AIBN in toluene was used depending on the used solvent. DMF = dimethyl formamide, NMP = *N*-methyl-2-pyrrolidone. Conversion determined *via* $^1$H NMR spectroscopy. $M_{n,theo.}$ in Table 2 calculated from $M_{n,theo.}$ = ([NAS] x $M_{NAS}$ x conversion) + $M_{CTA}$. $M_{n,GPC}$ in Table 3 determined via SEC in DMF and in Table 4 via GPC in DMF. In Tables 3 and 4 polydispersity Đ calculated from $M_{w,GPC}$/$M_{n,GPC}$. In Table 4 NAS:DAA ratio calculated from the $^1$H NMR spectrum and due to very high value Conv.$_{NAS}$ could not be obtained for entries 1 and 2.

**Table 2: Polymerization of NAS using a CTA1/RAFT**

| **Entry** | **Feed [NAS]/[CTA1]/ [AIBN]** | **Time [min]** | **Temp. [°C]** | **Solvent** | **Conv. [%]** | $M_{n,theo.}$ **[g/mol]** | $\mathbf{M_{n,NMR}}$ [g/mol] |
|---|---|---|---|---|---|---|---|
| 1 | 50/1/0.4 | 296 | 80 | DMF | 96 | 8500 | 18500 |
| 2 | 100/1/0.05 | 1392 | 80 | DMF | 75 | 13100 | 20000 |
| 3 | 50/1/0.05 | 298 | 80 | NMP | 39 | 3700 | 6000 |

**Table 3: Polymerization of NAS using a CTA2/RAFT**

| **Entry** | **Feed [NAS]/[CTA2]/[AIBN]** | **Time [h]** | **Temp. [°C]** | **Solvent** | **Conv. [%]** | $\mathbf{M_{n,GPC}}$. **[g/mol]** | **Đ.** |
|---|---|---|---|---|---|---|---|
| 1 | 50/1/0.05 | 23 | 80 | DMF | 67 | 7000 | 1.39 |
| 2 | 100/1/0.05 | 23 | 80 | DMF | 94 | n.d. | n.d. |
| 3 | 50/1/0.05 | 22.5 | 80 | NMP | 90 | 2900 | 1.40 |

**Table 4: Polymerization of NAS and DAA using CTA1.**

| Entry | Feed [NAS]/ [DAA]/ [CTA1]/ [AIBN] | Time [min] | Temp. [°C] | Solvent | Conv. NAS [%] | Conv. DAA [%] | $M_{n,GPC}$ [g/ mol] | Đ | NAS: DAA ratio |
|---|---|---|---|---|---|---|---|---|---|
| **1** | 25/25/1/0.1 | 1155 | 80 | DMF | - | 95 | 5100 | 1.46 | 52:48 |
| **2** | 50/25/1/0.1 | 1274 | 80 | DMF | - | 97 | 6200 | 1.60 | 65:35 |
| **3** | 25/50/1/0.1 | 1197 | 80 | DMF | 99 | 92 | 10485 | 1.18 | 35:65 |

**Example 2: Synthesis of P(SpAA) 1-3 and P(SpAA-co-DAA) 1-3**

Post-polymerization functionalization

Post-polymerization functionalization is a facile tool for access to pendant groups that are not easily accessible as monomers themselves. N-acryloxysuccinimide, an active ester of acrylic acid, was chosen as a monomer, because active esters are reactive towards nucleophiles in a straightforward addition-elimination reaction without generating toxic byproducts. After polymerization of NAS or copolymerization with DAA, the obtained homo- and copolymers were treated with one equivalent of tri-boc spermine 3 per NAS-repeating unit to convert NAS units to tri-boc spermine acrylamide (TBSpAA). To ensure full removal of active esters to prevent side reactions during in vivo and in vitro evaluations, the reaction mixture was treated with ammonia to convert unreacted NAS units to water-soluble acrylamides. After purification via precipitation from hexane and freeze-drying from benzene, the structure and purity of polymers was analyzed via NMR spectroscopy and SEC analysis.

Full conversion was proven by an absence of NAS signals in $^1$H NMR spectra. Signals could be assigned to either tri-boc spermine pendant group, DAA, or backbone protons. Additionally, no residual N-hydroxysuccinimide, which is formed during modification of P(NAS), is intercalated in the polymer, which was often observed if ammonia was not added. SEC analysis in DMF showed a shift in retention time towards higher molecular weights for all polymers. Molecular weights were not comparable between P(NAS) and P(TBSpAA) homo- and copolymers, due to the different interaction with the SEC column and broad polydispersities. $^1$H NMR spectroscopy was also used to determine the percentage of the tri-boc spermine TBSpAA and DAA in the obtained copolymers. The calculated ratio of spermine in P(TBSpAA-co-DAA) 1-3 showed different values than the NAS/DAA ratios (see Table 1). This is explained by the low amount of NAS groups that were converted into acrylamides instead of being functionalized by spermine leading to higher percentages of DAA in comparison to TBSpAA fractions.

Deprotection of polymers

Deprotection of all Boc-protected polymers was performed in trifluoroacetic acid (TFA) to obtain the TFA salts of the desired poly(spermine acrylamide) (P(SpAA)) and poly(spermine acrylamide-co-DAA) (P(SpAA-co-DAA)) polymers.

To compare the polymers to a small molecule with a similar structure (one primary and two secondary amines), tri-boc spermine 3 was converted to a propionyl-spermine amide species 4 in a two-step reaction, i.e., coupling to an activated propionyl followed by deprotection with trifluoroacetic acid. The resulting propionyl-spermine TFA-salt 4 was characterized via $^1$H and $^{13}$C NMR spectroscopy to help signal assignment of the poly(spermine acrylamide) polymers. $^1$H NMR spectra of the polymer TFA-salts showed similar chemical shifts of the spermine proton signals as 4 with a strong broadening of the peaks attributed to the polymeric structure. Additionally, the methylene group signal of 4 showed a similar chemical shift as the backbone protons of the isolated P(SpAA) polymers 1-3. $^{13}$C NMR spectra of the polymers showed the presence of TFA salt in the polymers, substantiating the assumption of polymer TFA-salts.

After deprotection, a final cationic to hydrophobic ratio (SpAA:DAA) of 83:17, 76:24 and 43:57 was obtained for the copolymers P(SpAA-co-DAA)1, P(SpAA-co-DAA)2 and P(SpAA co-DAA)3 as determined via $^1$H NMR spectroscopy. These values are in accordance with the ones before deprotection, showing that TFA treatment does not lead to any alteration of the polymer composition.

Buffering capacity

An important property of polycations is their buffering capacity to facilitate endosomal escape of siRNA. Therefore, the development of suitable carriers which induce escape of the genetic material from the endosome before it is degraded by lysosomes is a major task. Due to the buffering capacity of amine-containing polymers, endosomes can be destabilized due to a proton-sponge effect. The residual non-protonated amino groups of the polymers after complexation with siRNA are protonated due to a lower pH in the endosome causing the disruption of the membrane, release of the genetic material into the cytoplasm, and successful transfection. Branched poly(ethylenimine) shows a superior transfection efficiency, due to its high basic capacity caused by its specific polymer architecture since it is a branched polymer consisting of primary, secondary and tertiary amino groups exhibiting pKa values distributed over the entire physiological pH range acting as a proton sponge. The basic capacity of spermine-polymer P(SpAA)3 and spermine-molecule 4, both with a ratio of primary/secondary/tertiary amines = 1/2/0, was investigated by performing an acid/base titration with 0.1M HCl. It was compared to hyperbranched PEI with a molecular weight of 25 kg/mol and a ratio of primary/secondary/tertiary amines of 1/1.1/0.7 (given by manufacturer's method, BASF, Ludwigshafen, Germany). Additionally, spermine (primary/secondary/tertiary amines = 1/1/0) was investigated to compare the results to our polymers with respect to changes in basicity when one primary amine is converted to an amide. In these measurements, polymers with higher buffering capacities needed larger amounts of HCl for the alteration of the pH value of the solution. PEI has a substantial buffering capacity over almost the whole pH range. The buffering capacity at the pH values higher than 9 is mainly attributed to the primary and secondary amines with the reported pKa values of 8-9, whereas the buffering capacity at lower pH values, especially in the range of 5.5-7, might be associated to the tertiary amines with the pKa values of 6-7. Spermine itself is known to induce pH buffering. Since the same amount of substance (in mol) was used regarding each protonable unit, the buffering capacity of P(SpAA)3, 4 and spermine was found to be better than PEI in the region of pH 11 to pH 6. The P(SpAA)3 and the small modified spermine molecule 4 showed similar buffering capacities, with the latter performing slightly better, revealing the similarity of both structure with regards to the ratio of primary and secondary amines. Having the highest percentage of primary amines, spermine performed best in this region. Due to the absence of tertiary amines, a drop in pH value is observed for P(SpAA)3, 4 and spermine in the pH region of 5 to 2. These results suggest, that within the experimental pH range of 5-8, spermine polymers offer a buffering ability, which might facilitate endosomal escape.

Critical micelle concentration

The critical micelle concentration (CMC) is defined as the concentration of amphiphilic molecules required for the spontaneous formation of micelles. It is a key factor in the applications of amphiphilic polymers, including drug delivery, as it predicts the micelle-forming capacity and stability of polymeric micelles. Concentration series of P(SpAA)3 and P(SPAA-co-DAA) 1-3 in water were prepared. Using the water-insoluble Nile red, a fluorogenic dye that undergoes an increase of fluorescence intensity when encapsulated in the hydrophobic core of a micelle, CMC values were determined by plotting the concentration of the polymer versus the fluorescence of Nile red.

Homopolymer P(SpAA)3 was only slightly able to encapsulate small amounts of Nile Red when using concentrations of 0.1 mg/mL or more, but only a very low fluorescence was observed. It seems, that the hydrophobic backbone of the polymer leads to minor amphiphilic properties. In contrast, copolymers P(SPAA-co-DAA) 1-3 that bear different percentages of hydrophobic comonomer DAA can encapsulate considerable amounts of Nile Red. In detail, the CMC decreased from 0.24 mg/mL determined for P(SpAA-co-DAA)1 (17% DAA units) nearly six-fold to 0.041 mg/mL for P(SpAA-co-DAA)3 bearing 57% hydrophobic monomer units. This value is in the same range or even lower than hydrophobic PEI systems. In addition, not only the CMC decreased with increasing amounts of hydrophobic moieties, also the fluorescence value increased significantly indicating that higher amounts of Nile red could be encapsulated. Consequently, copolymers P(SPAA-co-DAA) 1-3 seemed to be promising candidates with amphiphilic properties that can enhance cellular uptake and stability of polyplexes.

**Example 3: Physico-chemical characterization of polyplexes with siRNA**

siRNA Encapsulation Ability

SYBR Gold assay was used to evaluate the capacity of the polymers to condense siRNA at various concentrations. An excellent condensation of siRNA is needed to protect siRNA to ensure cellular uptake while preventing enzymatic degradation. The synthesized positively charged polymers can electrostatically interact with negative charges of phosphate groups in siRNA which is quantified using the fluorescent dye SYBR Gold. Apart from P(SpAA)1-3 and P(SpAA-co-DAA), hyperbranched PEI (25 kg/mol) and spermine were used for comparison reasons. In a first comparison, the same concentration of polymers per siRNA were used, due to different protonable units, to compare polyplexes

prepared with the same amount of polymer (see **Fig. 3).** However, taking the higher molecular weight of the spermine-polymer repeating units into account, amine concentration is lower in comparison to PEI when using the same amount of polymer. All polymers and spermine were able to condense siRNA in which more efficient encapsulation was observed with increased concentrations, due to higher amounts of positively charged amines facilitating more electrostatic interactions with siRNA. Spermine showed a relatively low siRNA encapsulation ability with a constant value of approximately 4% free siRNA even at high concentrations attributed to the low molecular weight of spermine compared to the spermine homo- and copolymers. Homopolymers P(SpAA)1 and P(SpAA)3 showed full encapsulation at about 1.0 μg polymer per μg siRNA, whereas P(SpAA)2 showed a slightly better encapsulation. Due to the higher number of amines in PEI using same concentrations, siRNA encapsulation is more efficient leading to full encapsulation at weight ratios above 0.4 μgpolymer/μgsiRNA. In matters of N/P ratio this would be a value of 3 regarding the protonable unit of PEI which is in accordance with SYBR gold assays of hyperbranched PEI (25kDa). Regarding that commonly used number, P(SpAA)1-3 show full encapsulation already at N/P of 1, showing the excellent encapsulation ability of these polymers (see **Fig. 4).** An extensive use of free amine groups can be avoided that provokes high toxicities.

Copolymers P(SpAA-co-DAA)1 and P(SpAA-co-DAA)2 showed comparable siRNA profiles with a sufficient encapsulation of siRNA at concentrations of 1.0 μg polymer per μg siRNA. Furthermore, the P(SpAA-co-DAA)3 copolymer needed higher concentration for a complete encapsulation of siRNA due to high amounts of hydrophobic subunits that do not contribute to electrostatic interactions between amines and siRNA. siRNA encapsulation based on N/P ratio showed that also copolymers can encapsulate siRNA at an N/P ratio of one or higher (see **Fig. 4).** In the present example equation (1) for calculating the amount of polymer needed to obtain different N/P ratios described in detail elsewhere is:

$$\text{m (polymer in pg)} = \text{n siRNA (pmol)} \times \text{protonable unit (g/mol)} \times \text{N/P} \times \text{number of nucleotides siRNA.} \qquad \text{(eq. 1)}$$

It seems, that hydrophobic segments in the polymer do not negatively influence electrostatic interactions of siRNA and amines.

<u>Size and Zeta(ζ)-Potential Analysis</u>

Not only the full encapsulation of siRNA, but also size and charge of polyplexes play a major role in successful delivery of the payload. Particle sizes, polydispersity indices (PDI) and zeta potentials of polyplexes were measured using dynamic light scattering (DLS) and laser Doppler anemometry (LDA). Comparing polyplexes, formed with the same concentration of polymers using three different concentrations ($c_1$ = 5.603 μg/mL, $c_2$ = 7.844 μg/mL, $c_3$ = 11.206 μg/mL polymer representing N/P ratios of 5, 7 and 10 for PEI), all polyplexes were small with slightly positive zeta potentials indicating optimal siRNA encapsulation already at low concentrations low to moderate polydispersities. Solely, P(SPAA-co-DAA)3 was not able to fully encapsulate siRNA at a low concentration of $c_1$; which equals an N/P ratio of 0.75 for this polymer, shown by negative zeta-potentials and large, polydisperse particles. Since more phosphate groups are present than nitrogen atoms at such an N/P ratio, full condensations are rather uncommon. Additionally, spermine was not able to form polyplexes at all concentrations owing to its low molecular weight. siRNA that is not fully encapsulated and stuck on the surface of the polyplex causes negative zeta potentials as well as a detection of "free" siRNA as already shown for SYBR gold assays with spermine. Zeta-potentials of PEI-polyplexes were slightly positive to neutral at all concentrations even if those concentrations are already quite high for PEI in matters of N/P ratio (NP = 5 ($c_1$), 7 ($c_2$) and 10 ($c_3$)). All other spermine-polymers showed similar or higher zeta-potentials, showing that spermine-acrylamides were able to fully encapsulate siRNA already at low concentrations (equals N/P ratio of about 1) and higher concentrations (NP = 1.5 to 2.2). No correlation between sizes and zeta potentials with increasing concentrations could be found, as sizes remained small for all polyplexes with full siRNA encapsulation.

To further analyze sizes, polydispersities and zeta potentials with regard to the number of nitrogen atoms per polymer, measurements with all polymers were conducted at N/P ratios of 2, 5 and 7 which are in vitro relevant numbers (see Fig. 5). Since PEI does not show full encapsulation of siRNA at a N/P ratio of 2, zeta-potentials for these polyplexes were negative and polyplexes showed increased sizes and polydispersities. Polyplexes from spermine-acrylamides showed efficient siRNA encapsulation at low N/P ratios with small polyplexe sizes between 70 and 130 nm and positive zeta potentials (3 - 23 mV) indicating that these polymers can form optimal particles already at exceptionally low N/P ratios. Advantageously, the use of low amine concentrations helps avoiding unwanted side or toxic effects due to a low number of amines present in the system. Polyplexes from P(SpAA)1, P(SpAA)3, P(SPAA-co-DAA)1 and P(SPAA-co-DAA)2 formed at N/P ratios of 2 showed very slightly positive zeta potentials indicating a low amount of excess positive charges. With increasing N/P ratios, zeta potentials became more positive while sizes remained at around 100 nm for these polymers. For all polyplexes, polydispersities were low to moderate with values raging between 0.13 and 0.37,

with P(SPAA-co-DAA)3 forming the most monodisperse polyplexes (PDI = 0.14 - 0.23) at all tested N/P ratios, most likely due to its most amphiphilic character.

Stability of Polyplexes

To evaluate the polyplex stability in the presence of competing polyanions under neutral and acidic conditions, a heparin SYBR gold competition assay was performed to investigate the behavior in serum containing cell culture medium or for estimation of in vivo behavior. The ability of P(SpAA)1-3 and P(SpAA-co-DAA)1-3 for siRNA protection in the presence of increasing concentrations of polyanionic heparin under physiologically relevant conditions (pH 7.4) was tested (see **Fig. 6,** top). siRNA displacement at pH 7.4 from P(SpAA) polyplexes was not observed at low heparin concentrations and reached maximum release of less than 20% at high concentration of 1.00 I.U. heparin per well. P(SpAA)1 and P(SpAA)2 showed slightly better stability at pH 7.4 than P(SpAA)3 shown by a higher concentration of heparin necessary to replace initial amounts of siRNA. In comparison, PEI formed more loosely assembled polyplexes as higher amounts of siRNA (up to 30%) were released and already at low concentrations of heparin siRNA replacement took place. Similar observations were made for polyplexes with spermine at low heparin concentrations. At high concentrations of 1.00 I.U. heparin per well, a release of up to 80% siRNA was detected due to unstable polyplexes formed with spermine. P(SpAA-co-DAA) copolymers showed less effective siRNA complexation at higher concentration than homopolymers. Polyplexes from P(SpAA-co-DAA)2 and 3 started to release siRNA at a concentration of 0.25 whereas P(SpAA-co-DAA)1 formed more stable polyplexes with concentrations of 0.50 I.U. of heparin necessary to displace small amounts of siRNA. At high concentration all polyplexes from copolymers release between 35 and 55% siRNA. Even if this release is higher than for the homopolymers, similar values were observed for other polycationic systems.

Additionally, siRNA release ability under acidic conditions (pH 4.5), mimicking the endosomal compartment, was analyzed using a different buffer system (see **Fig. 6,** bottom). siRNA was more easily released from P(SpAA) and PEI polyplexes reaching almost 100% release for P(SpAA)2 and 70-90% for P(SpAA)1, P(SpAA)3 and PEI. A similar behavior was observed using copolymers, with all copolymers releasing 100% siRNA at high concentrations indicating suitable siRNA release abilities. Spermine is not able to form any stable polyplexes at pH 4.5. It seems, that amphiphilic copolymers P(SpAA-co-DAA) 1-3 and homopolymer 3 form less stable polyplexes which release siRNA even more efficient than PEI. These polyplexes might be less stable due to lower charge density or higher steric hindrance caused by the hydrophobic subunits, which in turn also helps to release the payload at lower pH values probably due to further destabilization caused by charge repulsion of additionally protonated amine groups.

**Example 4: In vitro performance of polyplexes in lung cells**

Cellular uptake in H1299 cells

Initial experiments indicated that spermine-containing polyacrylamides are suitable for siRNA delivery regarding siRNA encapsulation efficiency and physicochemical characteristics. To further test their ability to mediate internalization into H1299 human non-small cell lung carcinoma cells, quantification of the cellular uptake of Alexa Fluor 488-labeled siRNA by flow cytometry determining the median fluorescence intensity (MFI) was investigated. Incubation times of 24 h were chosen and polyplexes from P(SpAA) 1-3, P(SpAA-co-DAA)1-3 and PEI with fluorescent siRNA at N/P ratios of 2, 5 and 7 were analyzed. Trypan blue treatment, which was additionally applied in order to exclude extracellular fluorescent signals caused by cell surface-bound siRNA, resulted in insignificantly lower MFI values for all tested polyplexes, indicating that negligeable amounts of polyplexes were stuck to the outer cell membranes (see **Fig. 7** and **Fig. 8).**

For homopolymers (see **Fig. 7),** PEI polyplexes showed similar uptake abilities independent from the used N/P ratio. For P(SpAA) 1-3, the fluorescence signals of cellular internalized siRNA-AF488 decreased from N/P ratios of 2 to 7. Nonetheless, significantly higher uptake was reached at all N/P ratios in comparison to PEI-mediated siRNA uptake. Highest uptake was reached with P(SpAA)3 at N/P 2 which was about 6-times higher than PEI-mediated uptake experiments. P(SpAA)1 still induced more than three-fold cellular uptake even at N/P 7, showing that all P(SpAA) homopolymers are able to outperform commonly used PEI at all tested N/P ratios. Similar trends were observed for copolymers P(SpAA-co-DAA)1-3 (see **Fig. 8).** All polyplexes induced higher cellular uptake in comparison to PEI-polyplexes with a dependency on the hydrophobic fraction of the polymer on the MFI, however without a clear influence of the N/P ratio. The copolymer with the lowest hydrophobic ratio (P(SpAA-co-DAA) 1) induced between 3 and 4-times higher uptake compared to PEI polyplexes, which is in the same range as the homopolymers. With higher hydrophobic fraction, copolymers further enhanced cellular uptake of Alexa Fluor 488-labeled siRNA. P(SpAA-co-DAA)2 polyplexes formed at N/P 2 and 5 nearly reached performance of positive control Lipofectamin (LF) without being significantly different to LF uptake experiments. Remarkably, P(SpAA-co-DAA)3 polyplexes formed at N/P 2 were able to outperform "gold standard" Lipofectamin and additionally induced 10-times higher cellular uptake of siRNA in comparison to PEI.

eGFP Knockdown in H1299 cells

To evaluate the gene silencing efficiency of spermine-acrylamide polyplexes on the protein level, H1299/eGFP cells were utilized that express the 'enhanced green fluorescent protein' reporter gene (eGFP). Based on in vitro cellular uptake experiments, H1299/eGFP cells were transfected with PEI, P(SpAA)2, P(SpAA-co-DAA)2 or P(SpAA-co-DAA)3 polyplexes formulated with siRNA against eGFP (siGFP) or with scrambled siRNA (siNC) as negative control using N/P ratios of 2, 5 or 7 to determine optimal parameters for efficient protein knockdown (see Fig. 9). As positive control, Lipofectamin 2000 lipoplexes were used. After treatment, the median fluorescence intensity of eGFP in each sample was quantified via flow cytometry after 48 hours of incubation. Polyplexes containing siNC did not reduce the MFI of GFP, proving that protein knockdown not attributed to the polymeric system or any non-specific effects. All siGFP-polyplexes from spermine-polyplexes were able provoke eGFP knockdown between 20 and 47%, however with P(SpAA-co-DAA)2 polyplexes at N/P 5 showing statistically insignificant knockdown ability. Whereas PEI solely showed a significant knockdown at N/P ratios of 5 and 7 with up to 36% eGFP knockdown, P(SpAA-co-DAA)3 showed a constant knockdown after 48h of 47 to 40% at all tested N/P ratios even without chloroquine treatment. These findings emphasize that P(SpAA-co-DAA)3 polyplexes efficiently escape the endosome. Consequently, polyplexes with P(SpAA-co-DAA)3 can be used at various N/P ratios without drastic effects on their performance allowing less optimization steps and thus also reduced costs.

Staining and GAPDH Knockdown in ALI culture

Following validation of cellular uptake and reporter protein silencing mediated by poly(spermine acrylamide)-polyplexes in vitro, further evaluation of uptake and knockdown abilities in a more in vivo relevant context was performed using air-liquid interface (ALI) cultures. Mucus staining of Calu3 cells at ALI cultures was performed by using WGA-AF488 staining of mucus layer. Polyplexes were prepared using P(SpAA-co-DAA)3 and red fluorescent AF647 siRNA at an N/P ratio of 5. After 24 hours of incubation time, AF647-siRNA can be found below the mucus layer indicating that polyplexes can overcome the mucus barrier. Cell uptake was visualized by staining of Calu3 Cells and siRNA (see **Fig. 10, left** and **Fig.** 11). Nuclei were stained by DAPI (blue) and the cytoskeleton was stained using phalloidin. An incubation time of 24 hours and the same polyplexes as for mucus staining were used. Confocal microscopy confirmed that siRNA was inside the cells, located in the cytoplasm of Calu3 cells confirming cellular uptake of polyplexes. Thus, in vitro 2D and 3D cell culture experiments and in vivo experiments revealed superior properties of amphiphilic P(SpAA-co-DAA) in delivery of siRNA to lung cells showing a perfect balance between polyplex formation, toxicity, and siRNA delivery efficiency.

The ability of spermine-acrylamides to silence the endogenously expressed housekeeping gene GAPDH in Calu3 (human lung cancer) monolayers, after overcoming mucus barrier and cellular uptake, was additionally tested. Cells were transfected with P(SpAA-co-DAA)3 polyplexes containing GAPDH siRNA (siGAPDH) or scrambled sequence siRNA (siNC as negative control) at N/P 5 for 24 h. Negative controls consisted of blank cells that were treated with 5% glucose only while positive control cells were transfected with Lipofectamine 2000. GAPDH gene expression was normalized to $\beta$-actin gene expression and quantified by real time PCR (see **Fig. 10, right).** Calu3 cells that were transfected with P(SpAA-co-DAA)3/siGAPDH polyplexes showed higher levels of gene knockdown when compared to the blank cells or those that were treated with P(SpAA-co-DAA)3/siNC polyplexes. Lower levels of reduced GAPDH gene expression were also observed in comparison to cells that were treated with LF/siGAPDH indicating that P(SpAA-co-DAA)3 polyplexes are able to efficiently deliver siRNA resulting in efficient gene knockdown in vitro. In more sophisticated models such as ALI cultures, especially when mucus is present, lipofectamine is shown not to be as effective as in standard in vitro experiments, while polyplexes from P(SpAA-co-DAA)3 can still efficiently mediate a target gene downregulation. Additionally, Lipofectamin cannot be employed in in vivo experiments due to its toxicity, thus P(SpAA-co-DAA)3 is a conceivable alternative for safe and efficient siRNA delivery to lung cells via pulmonary delivery routes.

**Example 5: Toxicity experiments**

Thiazolyl blue tetrazolium bromide (MTT) assays (see Fig. 12A) were used to colorimetrically evaluate cell proliferation as well as the viability of the cells of all spermine-containing homo- and copolymers and PEI on the L929 mouse fibroblasts cell line after 24 h of incubation using different polymer concentrations. Results are presented as the percentage of cell viability compared to untreated control cells. IC50 (half-inhibitory concentration) values were calculated by plotting cell viability and concentration of polymers on a logarithmic scale using a sigmoidal model fit (see Fig. 12B). All spermines polymers affected the cell viability less than PEI (25 kg/mol, IC50 = 19.05 $\mu$g/mL) and IC50 values were between 25.40 to 71.49 $\mu$g/mL. P(SpAA-co-DAA)3 showed the lowest toxicity with regards to the IC50 value, most likely due to the lower number of free amine groups when using the same concentrations of polymers. Especially considering the facts that all spermine-polymers had much higher molecular-weights than the tested PEI and that high molecular-weight polymers are considered to be more toxic, lowering of the molar mass of poly(spermine acrylamide) homo- and copolymers can have a beneficial effect on their biocompatibility.

To further test the toxicity of the newly developed polycationic polymers in a more relevant setup when used for pulmonary siRNA delivery, immune and cytokine responses of polyplexes from PEI and P(SpAA-co-DAA)3 at an N/P ratio of 5 was analyzed in mice. Bronchoalveolar lavage fluid (BALF) and BALF cells were collected after mice were intratracheally treated with PEI or P(SpAA-co-DAA)3/siRNA polyplexes for determining concentration of various cytokines (see **Fig. 13).** For most of the tested cytokines, P(SpAA-co-DAA)3 treated mice showed similar or lower concentrations than untreated mice (IL-23, IL-27, IFN-y, IL-12p70, IL-10, IFN-b) or the values were even below the detection limit (IL-1a, GM-CSF). Solely, IL-1b, IL-6, TNF-a and IL-17A concentrations were slightly, but not significantly higher after P(SpAA-co-DAA)3 polyplex treatment and MCP-1 concentration showed a 6-fold increase. PEI/siRNA complexes showed similar proinflammatory effects, with an increased IL-23 level and without increasing MCP-1 concentration. As for some cytokines, concentrations were even lower than PEI, which generally shows very low levels of cytokines, following that the spermine-polymers are a safe and biocompatible delivery system.

## Claims

1. A process for the synthesis of poly(spermine acrylamide) and/or poly(spermine acrylamide-co-*N*-alkylacrylamide), the process comprising the following steps:

   a) Reacting a mixture of *N*-acryloxysuccinimide with *N*-alkylacrylamide, and
   b) Obtaining poly(*N*-acryloxysuccinimde) polymers, poly(*N*-alkylacrylamide) polymers and/or copolymers with varying ratios of N-acryloxysuccinimide and *N*-alkylacrylamide, and
   c) Treating the obtained polymers and/or copolymers of step b) with at least 0.1 equivalent of tri-boc spermine per *N*-acryloxysuccinimide-repeating unit and obtaining poly(spermine acrylamide) and/or poly(spermine acrylamide-co-*N*-alkylacrylamide) polymers.

2. The process according to claim 1, wherein the reacting in step a) is via free radical polymerization using a polymerization initiator and/or via reversible addition-fragmentation chain transfer polymerization using a chain-transfer agent and a polymerization initiator.

3. The process according to claim 1 or 2, wherein in step c) at least one of the amine groups is protected using ethyl trifluoroacetate and/or di-tert-butyl dicarbonate.

4. The process according to any of the claim 1 to 3, wherein in step c) a deprotection of the amine groups, preferably using an acid, is perfomed.

5. The process according to any of the claims 1 to 4, wherein in step c) ammonia is added.

6. The process according to any of the claims 2 to 4, wherein the polymerization initiator is azobisisobutyronitrile and/or a solvent is used wherein the solvent is dioxane, tetrahydrofuran, pyridine, dimethyl formamide, *N*-methyl-2-pyrrolidone and/or toluene.

7. A nanocarrier comprising a non-viral delivery agent and at least one substance, wherein the non-viral delivery agent is poly(spermine acrylamide) and/or poly(spermine acrylamide-co-*N*-alkylacrylamide), preferably produced by a process according to any of the claims 1 to 6.

8. The nanocarrier according to claim 7, wherein the nanocarrier comprises at least one substance, wherein the non-viral delivery agent is forming a complex, preferably a polyplex, with the substance.

9. The nanocarrier according to claim 7 or 8, wherein the substance is a nucleic acid and/or a nucleic acid dye.

10. A nanocarrier according to any of the claim 7 to 9 and/or a pharmaceutical composition comprising a nanocarrier according to any of the claim 7 to 9 and at least one further ingredient for use as a medicament for preventing the development, for slowing the progression and/or in the treatment of a disease and/or disorder, in particular a mental, neurodegenerative and/or neurological disease and/or disorder, a viral and/or bacterial infection, a respiratory or pulmonary disease, a metabolic disease, a metabolic syndrome and/or cancer.

**11.** A nanocarrier according to any of the claim 7 to 9 and/or a pharmaceutical composition comprising a nanocarrier according to any of the claim 7 to 9 and at least one further ingredient for use in the treatment of a disease and/or disorder, in particular a mental, neurodegenerative and/or neurological disease and/or disorder, a viral and/or bacterial infection, a respiratory or pulmonary disease, a metabolic disease, a metabolic syndrome and/or cancer.

**12.** A nanocarrier according to any of the claim 7 to 9 and/or a pharmaceutical composition comprising a nanocarrier according to any of the claim 7 to 9 and at least one further ingredient for use in a thera-peutic procedure or in a non-therapeutic procedure for preventing the development, for slowing the progression and/or for the treatment of a disease and/or disorder, in particular a mental, neurodegenerative and/or neurological disease and/or disorder, a viral and/or bacterial infection, a respiratory or pulmonary disease, a metabolic disease, a metabolic syndrome and/or cancer.

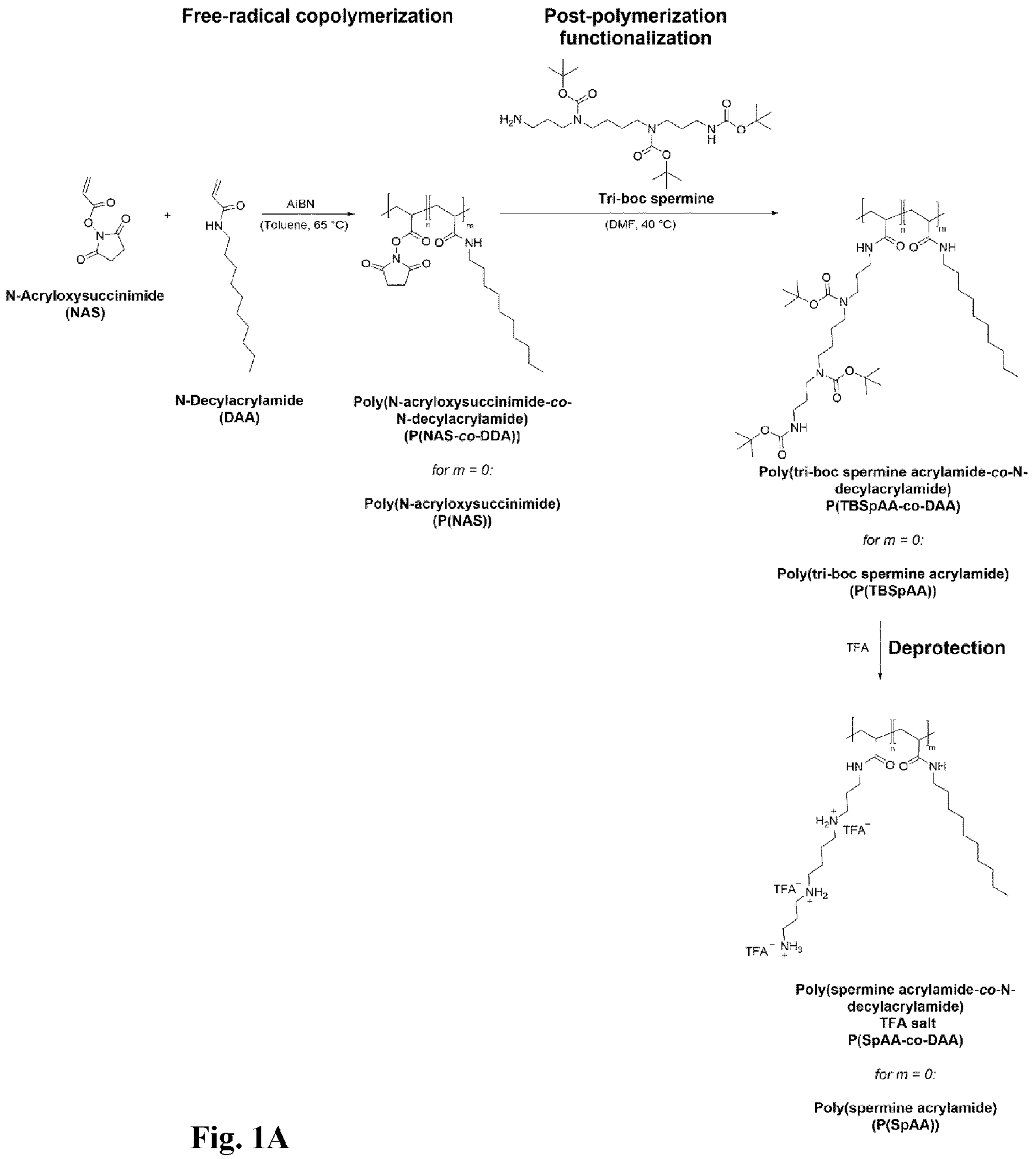


N-Acryloxysuccinimide (NAS)

N-Decylacrylamide (DAA)

Poly(N-acryloxysuccinimide-*co*-N-decylacrylamide) (P(NAS-*co*-DDA))

*for m = 0:*

Poly(N-acryloxysuccinimide) (P(NAS))

Poly(tri-boc spermine acrylamide-*co*-N-decylacrylamide) P(TBSpAA-co-DAA)

*for m = 0:*

Poly(tri-boc spermine acrylamide) (P(TBSpAA))

Poly(spermine acrylamide-*co*-N-decylacrylamide) TFA salt P(SpAA-co-DAA)

*for m = 0:*

Poly(spermine acrylamide) (P(SpAA))

Fig. 1A

**Free-radical polymerization / RAFT polymerization (CTA)** **Post-polymerization functionalization** **Deprotection**

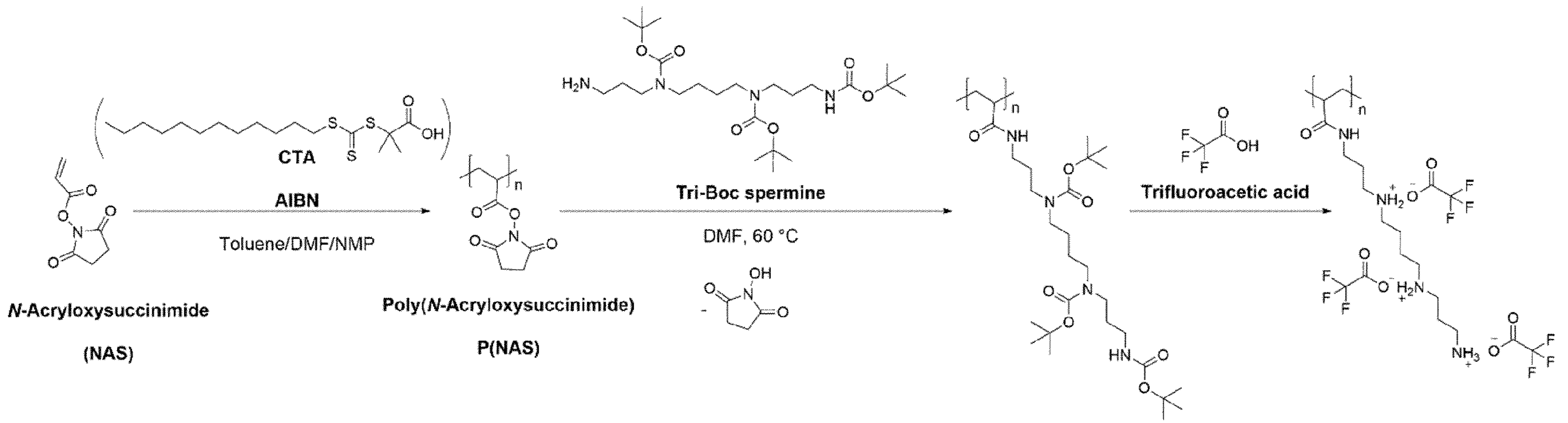


*N*-Acryloxysuccinimide (NAS)

Poly(*N*-Acryloxysuccinimide) P(NAS)

Poly(Tri-Boc spermine acrylamide) P(TBSpAA)

Poly(Spermine acrylamide) TFA salt P(SpAA) TFA salt

**Fig. 1B**

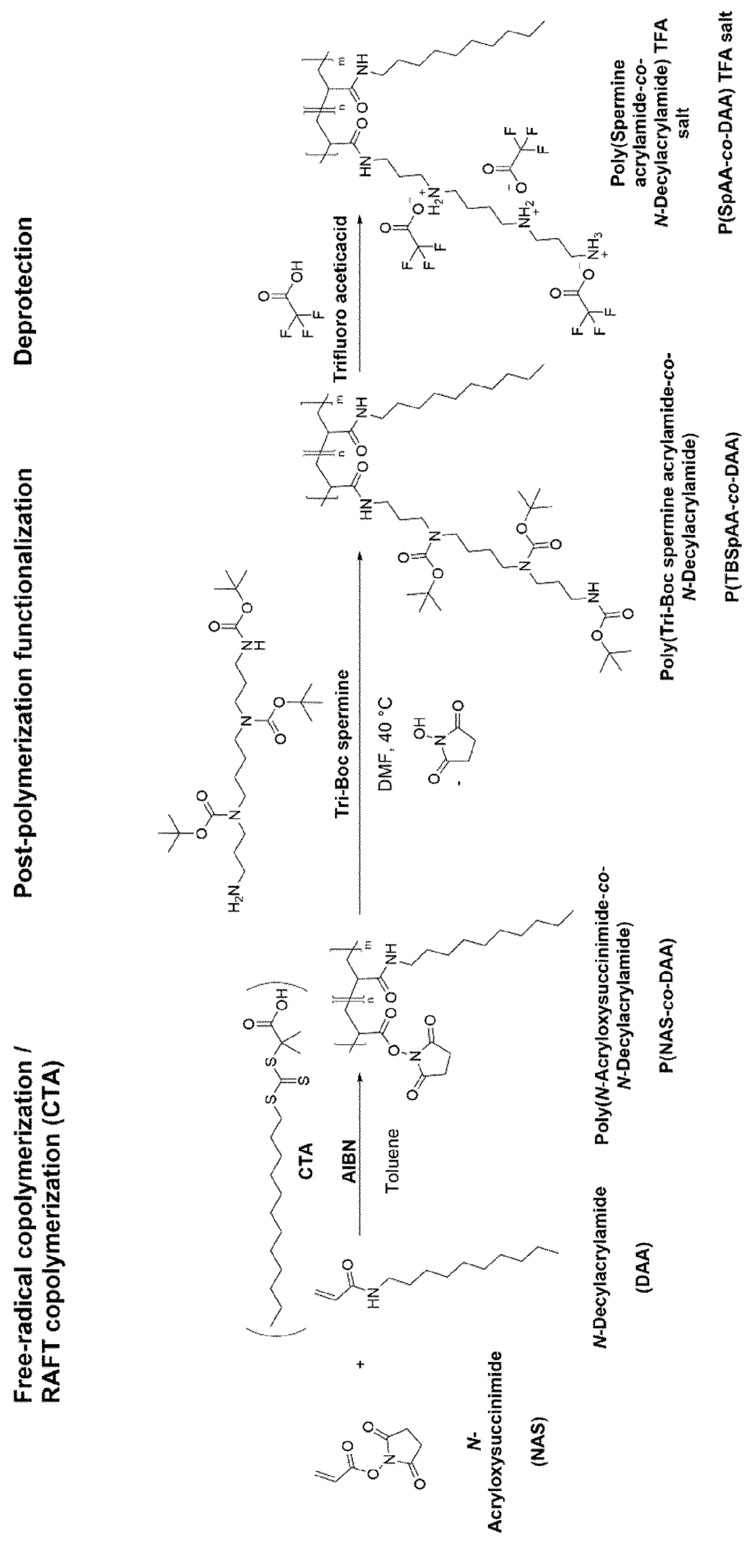

Free-radical copolymerization /
RAFT copolymerization (CTA)
Post-polymerization functionalization
Deprotection
CTA
AIBN
Toluene
Tri-Boc spermine
DMF, 40 °C
Trifluoro aceticacid
*N*-Acryloxysuccinimide
(NAS)
*N*-Decylacrylamide
(DAA)
Poly(*N*-Acryloxysuccinimide-co-*N*-Decylacrylamide)
P(NAS-*co*-DAA)
Poly(Tri-Boc spermine acrylamide-*co*-*N*-Decylacrylamide)
P(TBSpAA-*co*-DAA)
Poly(Spermine acrylamide-co-*N*-Decylacrylamide) TFA salt
P(SpAA-*co*-DAA) TFA salt


Fig. 1C

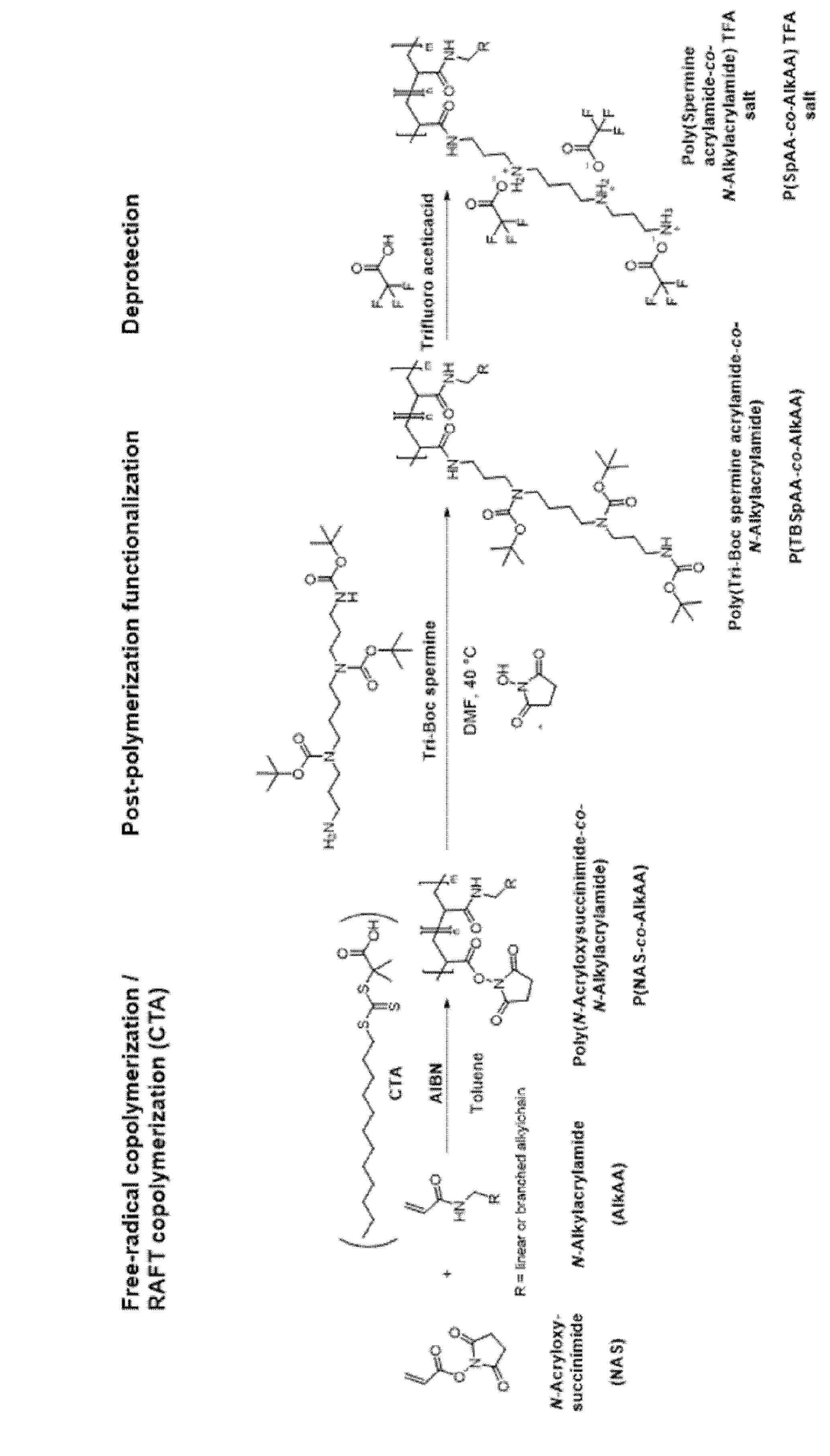
Free-radical copolymerization /
RAFT copolymerization (CTA)
Post-polymerization functionalization
Deprotection
N-Acryloxy-
succinimide
(NAS)
N-Alkylacrylamide
(AlkAA)
R = linear or branched alkylchain
CTA
AIBN
Toluene
Poly(N-Acryloxysuccinimide-co-
N-Alkylacrylamide)
P(NAS-co-AlkAA)
Tri-Boc spermine
DMF, 40 °C
Poly(Tri-Boc spermine acrylamide-co-
N-Alkylacrylamide)
P(TBSpAA-co-AlkAA)
Trifluoro aceticacid
Poly(Spermine
acrylamide-co-
N-Alkylacrylamide) TFA
salt
P(SpAA-co-AlkAA) TFA
salt

**Fig. 1D**

**short alkyl chains:**

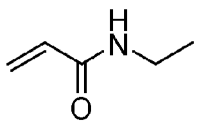

n = 1

N-Ethylacrylamide

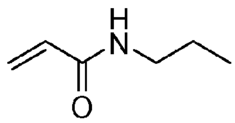

n = 2

N-Propylacrylamide

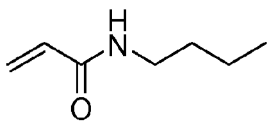

n = 3

N-Butylacrylamide

**long alkyl chains:**

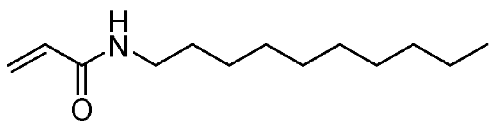

n = 9

N-Decylacrylamide

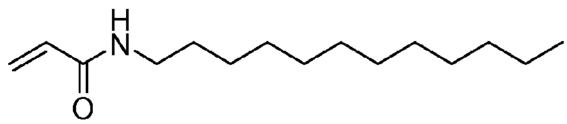

n = 11

N-Dodecylacrylamide

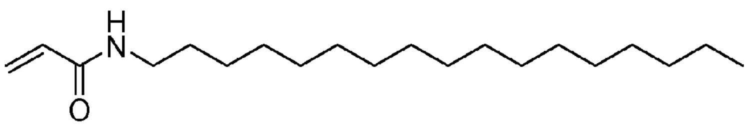

n = 16

N-Hexadecylacrylamide

**branched alkyl chains:**

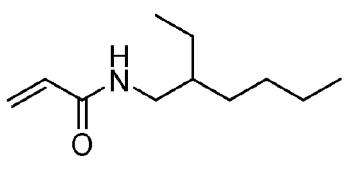

m = 1, o = 1, p = 3

N-2-Ethylhexylacrylamide

**Fig. 1E**

Accessible via free radical polymerization:

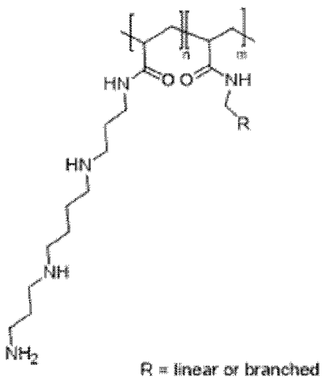

R = linear or branched alkyl chain

**Poly(Spermine acrylamide-*co*-*N*-Alkylacrylamide)**

**P(SpAA-*co*-AlkAA)**

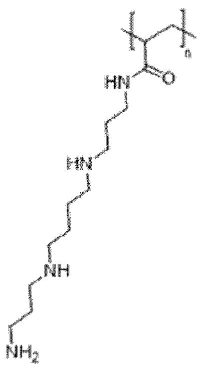

**Poly(Spermine acrylamide)**

**P(SpAA)**

Accessible via RAFT:

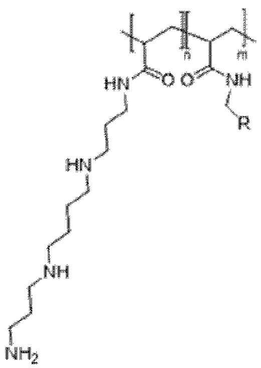

**Poly(Spermine acrylamide-*co*-*N*-Alkylacrylamide)**

**P(SpAA-*co*-AlkAA)**
[Statistical Copolymer or Blockcopolymer]

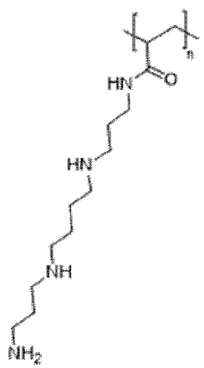

**Poly(Spermine acrylamide)**

**P(SpAA)**

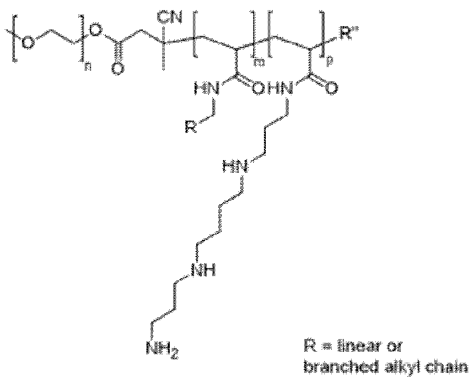

R = linear or branched alkyl chain

**Methoxy-poly(ethylene-glycol)-*block*-poly(Spermine acrylamide-*co*-*N*-Alkylacrylamide)**

**mPEG-*b*-P(SpAA-co-AlkAA)**
[Statistical Copolymer or Blockcopolymer]

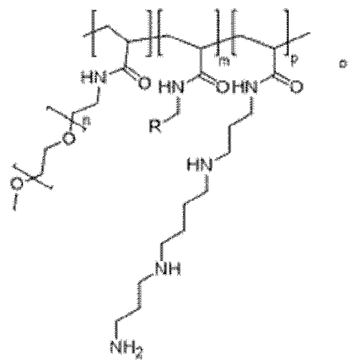

**Poly(methoxyPEG-*co*-Spermine acrylamide-co-N-Alkylacrylamide)**

**P(mPEG-*co*-PSpAA-*co*-AlkAA)**
[Statistical Copolymer or Blockcopolymer]

Fig. 1F

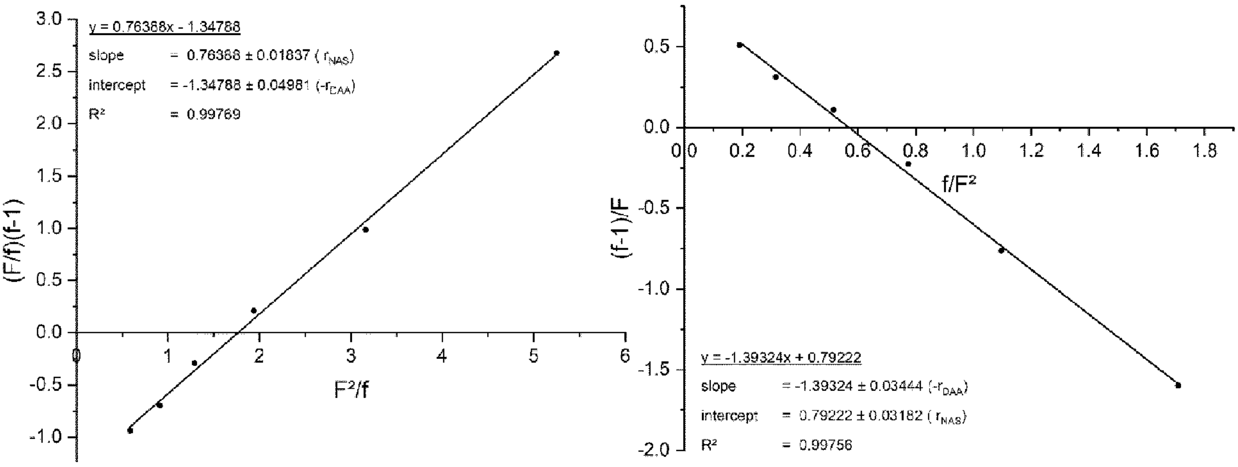
y = 0.76368x - 1.34788
slope = 0.76368 ± 0.01837 ( r_NAS)
intercept = -1.34788 ± 0.04981 (-r_CAA)
R² = 0.99769
(F/f)(f-1)
F²/f
y = -1.39324x + 0.79222
slope = -1.39324 ± 0.03444 (-r_DAA)
intercept = 0.79222 ± 0.03182 ( r_NAS)
R² = 0.99756
(f-1)/F
f/F²

**Fig. 2**

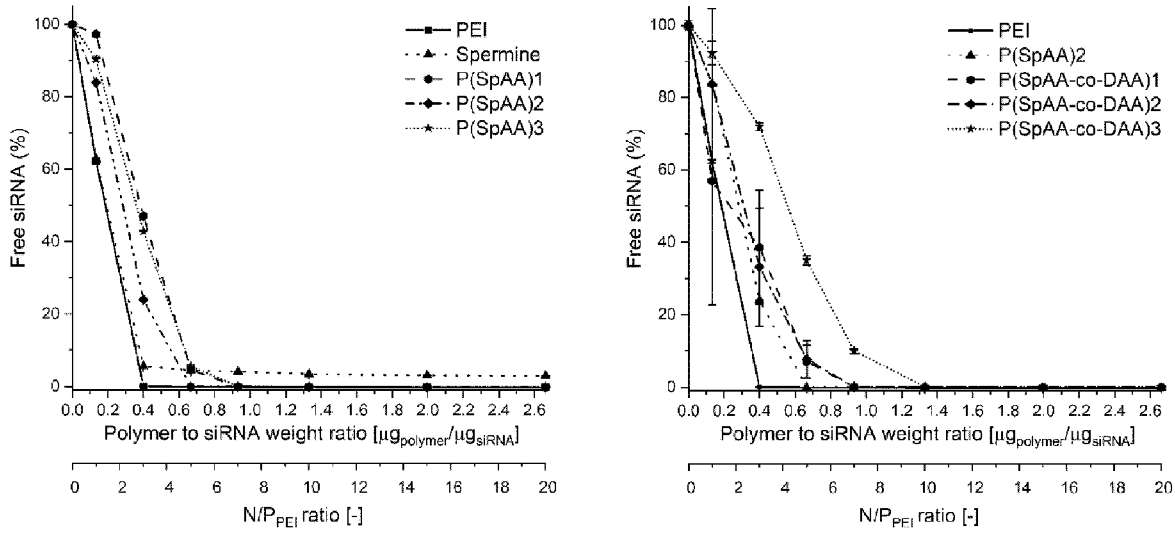
PEI
Spermine
P(SpAA)1
P(SpAA)2
P(SpAA)3
P(SpAA-co-DAA)1
P(SpAA-co-DAA)2
P(SpAA-co-DAA)3
Free siRNA (%)
Polymer to siRNA weight ratio [µg_polymer/µg_siRNA]
N/P_PEI ratio [-]

**Fig. 3**

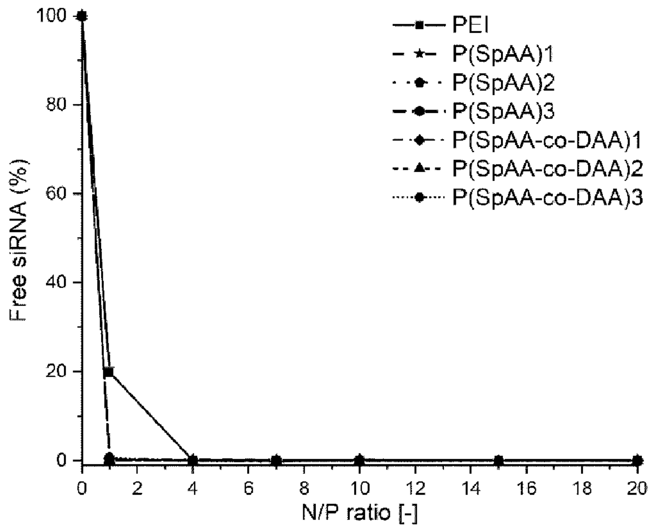
Free siRNA (%)
N/P ratio [-]
PEI
P(SpAA)1
P(SpAA)2
P(SpAA)3
P(SpAA-co-DAA)1
P(SpAA-co-DAA)2
P(SpAA-co-DAA)3

Fig. 4

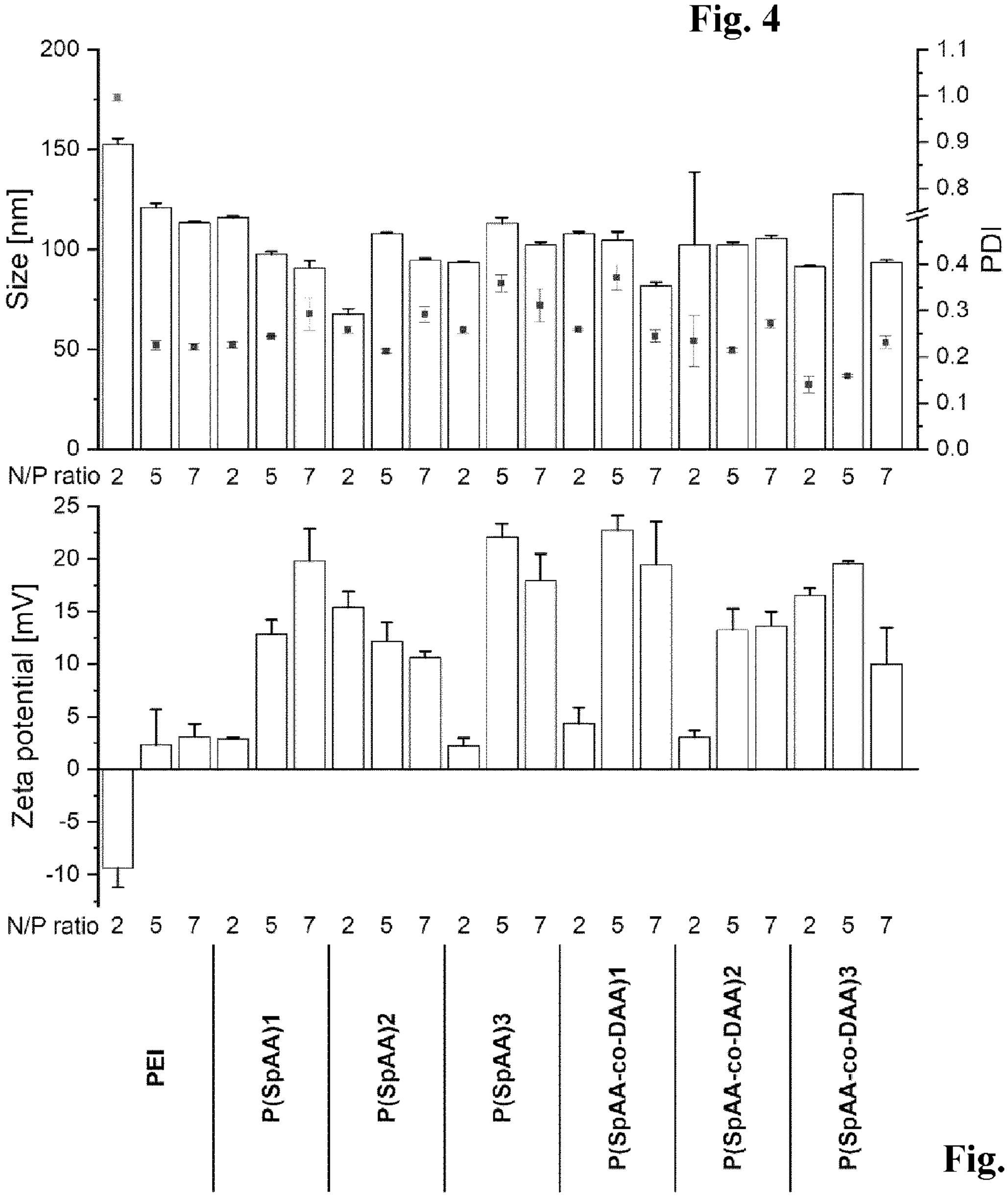
Size [nm]
PDI
N/P ratio
Zeta potential [mV]
PEI
P(SpAA)1
P(SpAA)2
P(SpAA)3
P(SpAA-co-DAA)1
P(SpAA-co-DAA)2
P(SpAA-co-DAA)3

Fig. 5

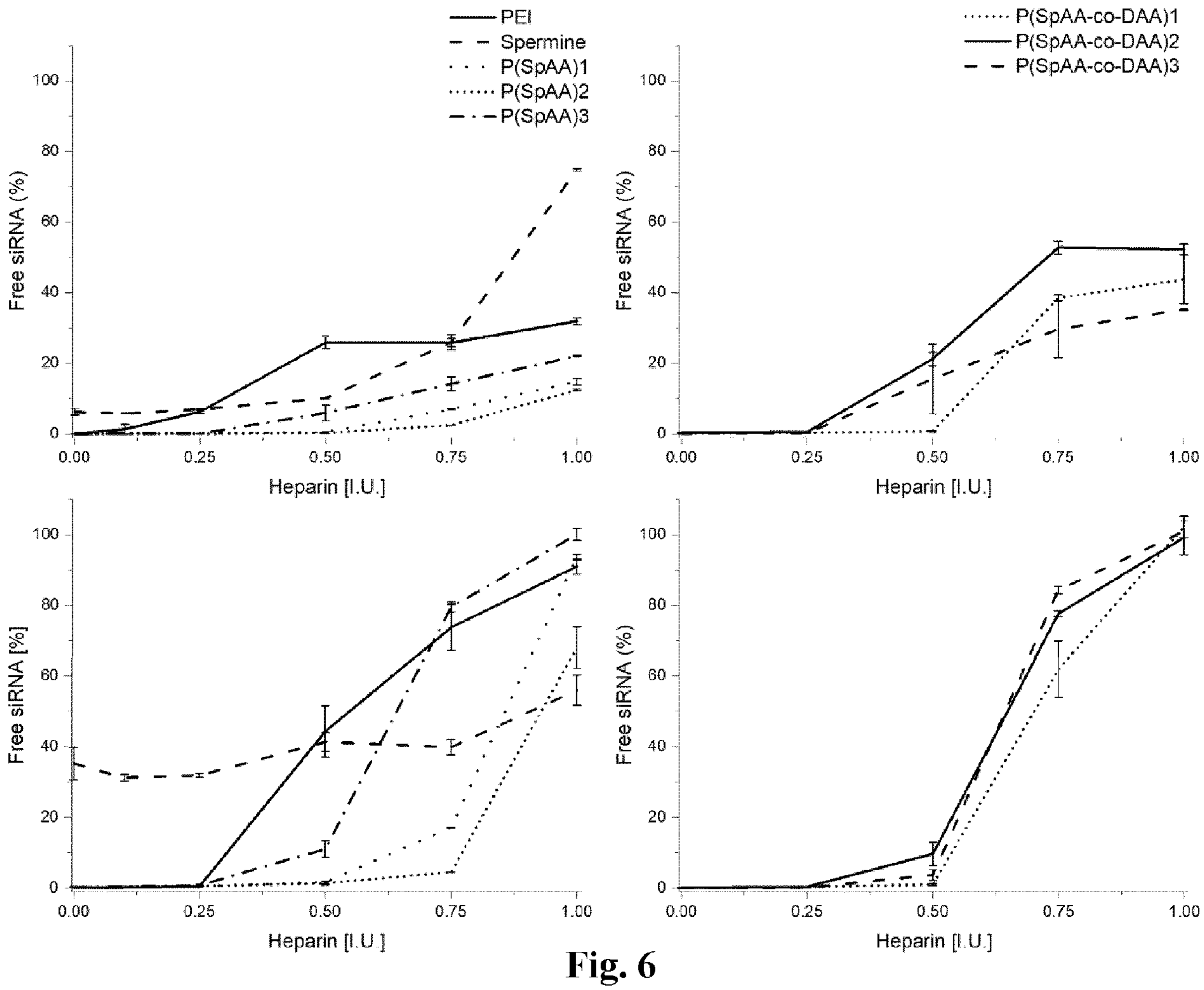
PEI
Spermine
P(SpAA)1
P(SpAA)2
P(SpAA)3
P(SpAA-co-DAA)1
P(SpAA-co-DAA)2
P(SpAA-co-DAA)3
Free siRNA (%)
Free siRNA [%]
Heparin [I.U.]
0.00
0.25
0.50
0.75
1.00
0
20
40
60
80
100

**Fig. 6**

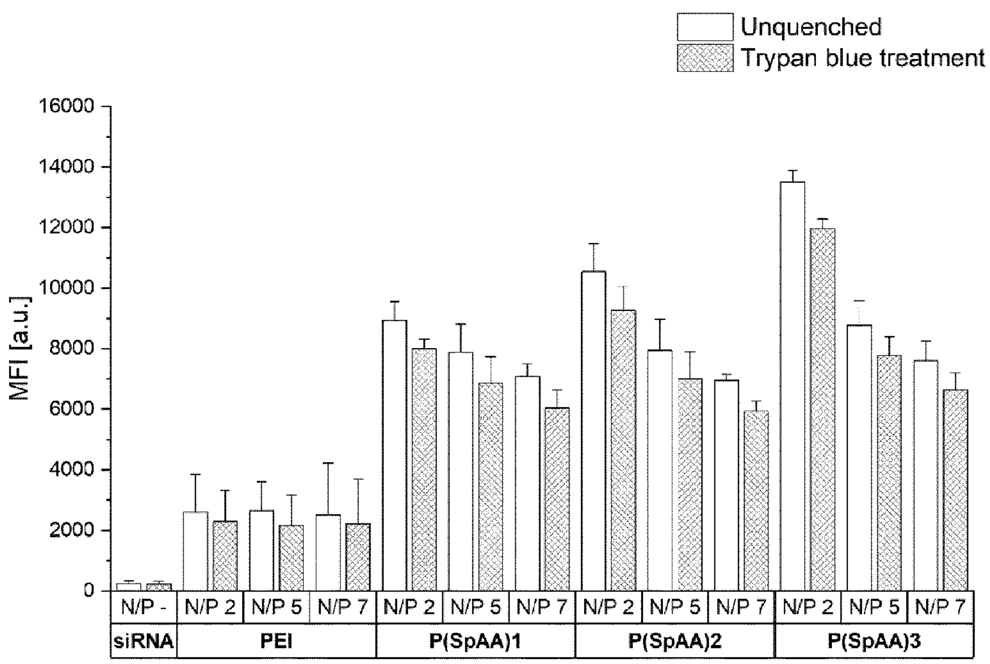
Unquenched
Trypan blue treatment
MFI [a.u.]
0
2000
4000
6000
8000
10000
12000
14000
16000
N/P -
N/P 2
N/P 5
N/P 7
siRNA
PEI
P(SpAA)1
P(SpAA)2
P(SpAA)3

**Fig. 7**

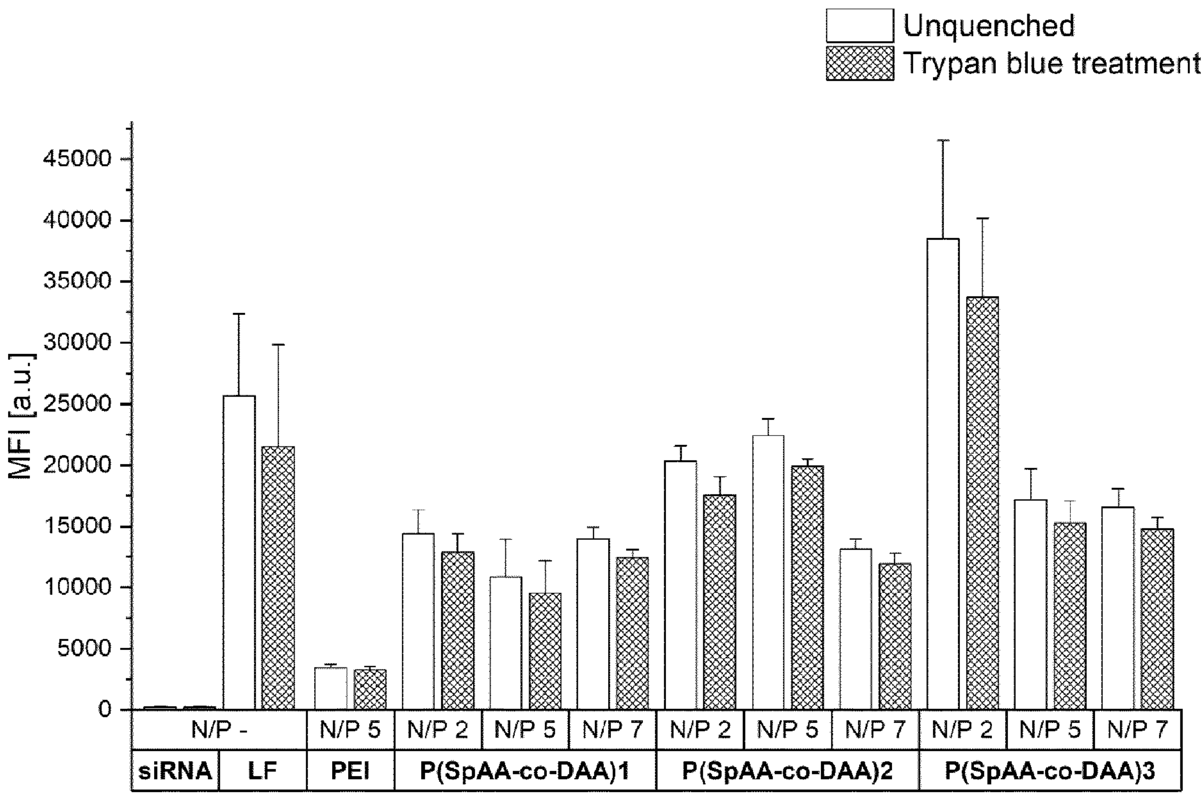
Unquenched
Trypan blue treatment
MFI [a.u.]
45000
40000
35000
30000
25000
20000
15000
10000
5000
0
N/P -
N/P 5
N/P 2
N/P 5
N/P 7
N/P 2
N/P 5
N/P 7
N/P 2
N/P 5
N/P 7
siRNA
LF
PEI
P(SpAA-co-DAA)1
P(SpAA-co-DAA)2
P(SpAA-co-DAA)3

**Fig. 8**

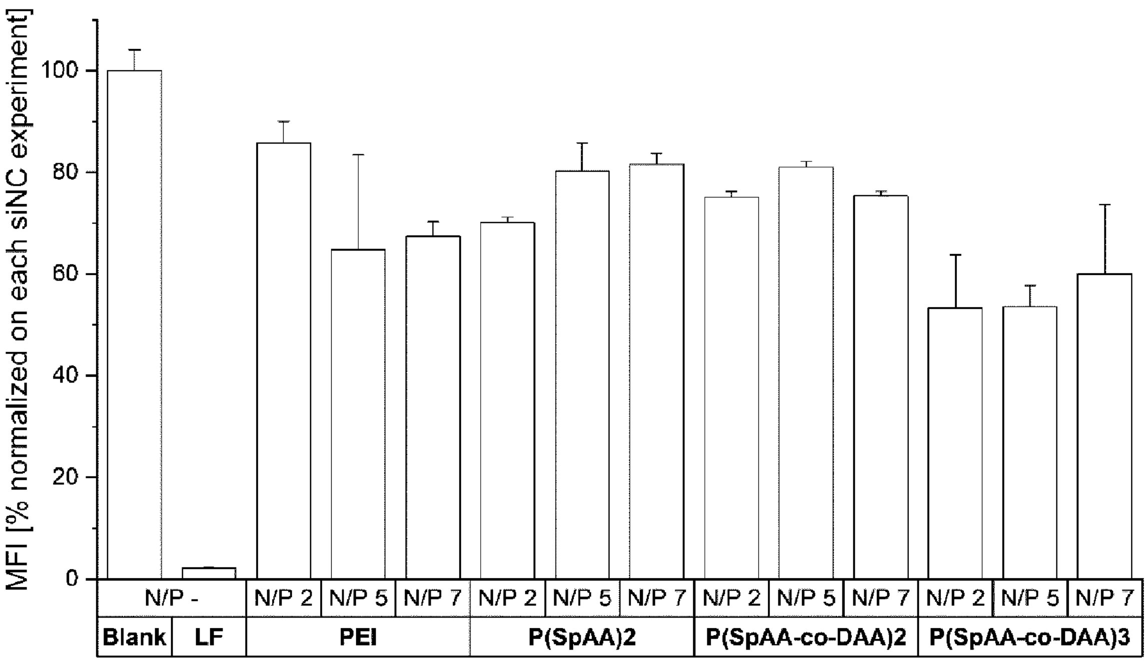
MFI [% normalized on each siNC experiment]
100
80
60
40
20
0
N/P -
N/P 2
N/P 5
N/P 7
N/P 2
N/P 5
N/P 7
N/P 2
N/P 5
N/P 7
N/P 2
N/P 5
N/P 7
Blank
LF
PEI
P(SpAA)2
P(SpAA-co-DAA)2
P(SpAA-co-DAA)3

**Fig. 9**

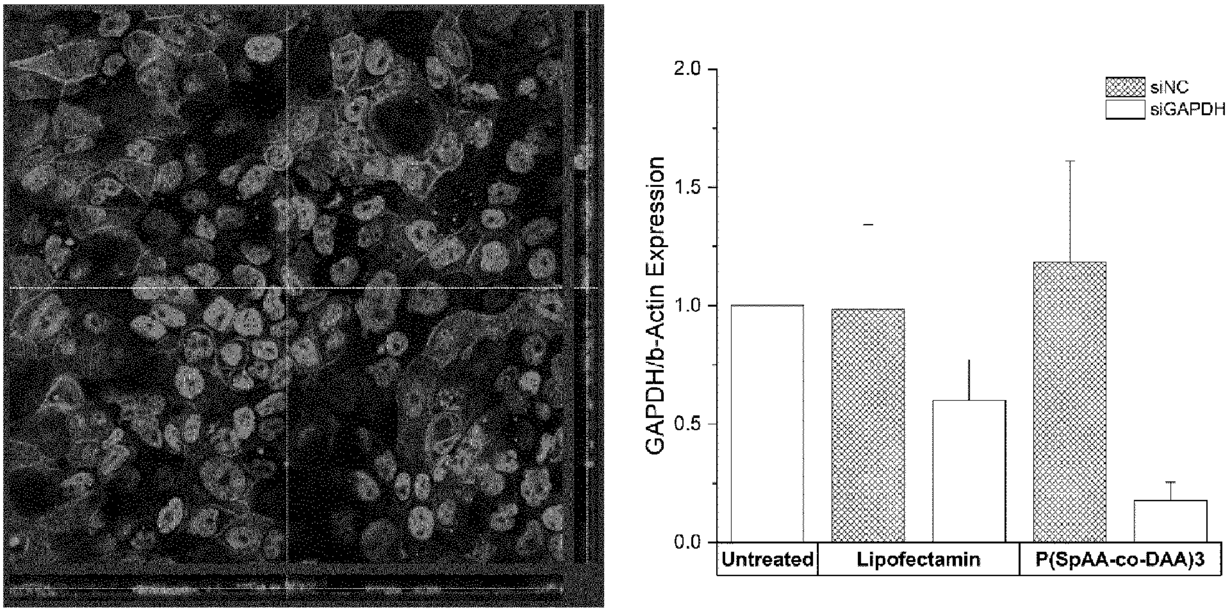
2.0
1.5
1.0
0.5
0.0
GAPDH/b-Actin Expression
siNC
siGAPDH
Untreated
Lipofectamin
P(SpAA-co-DAA)3

Fig. 10

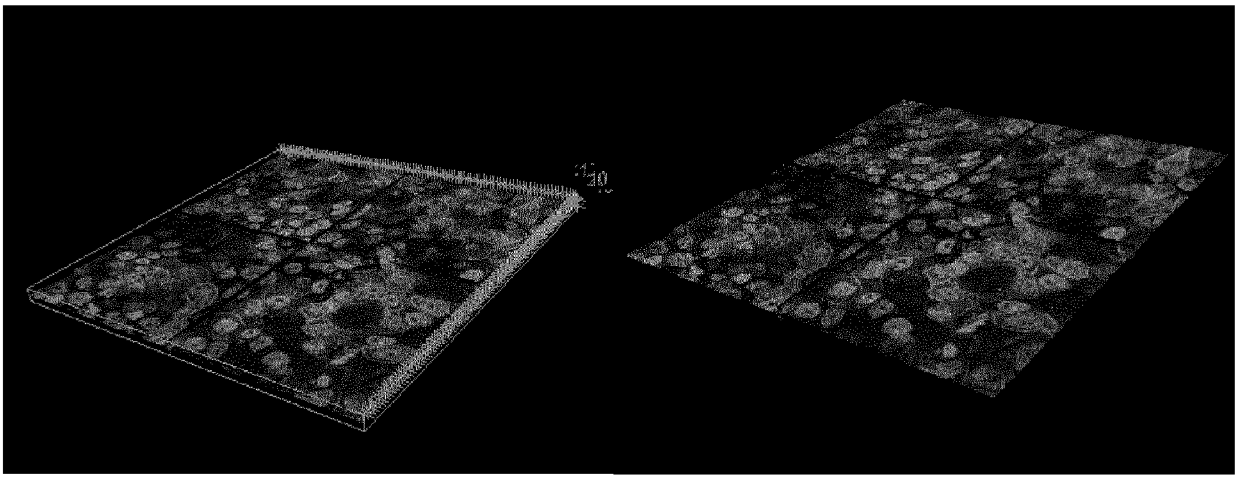

Fig. 11

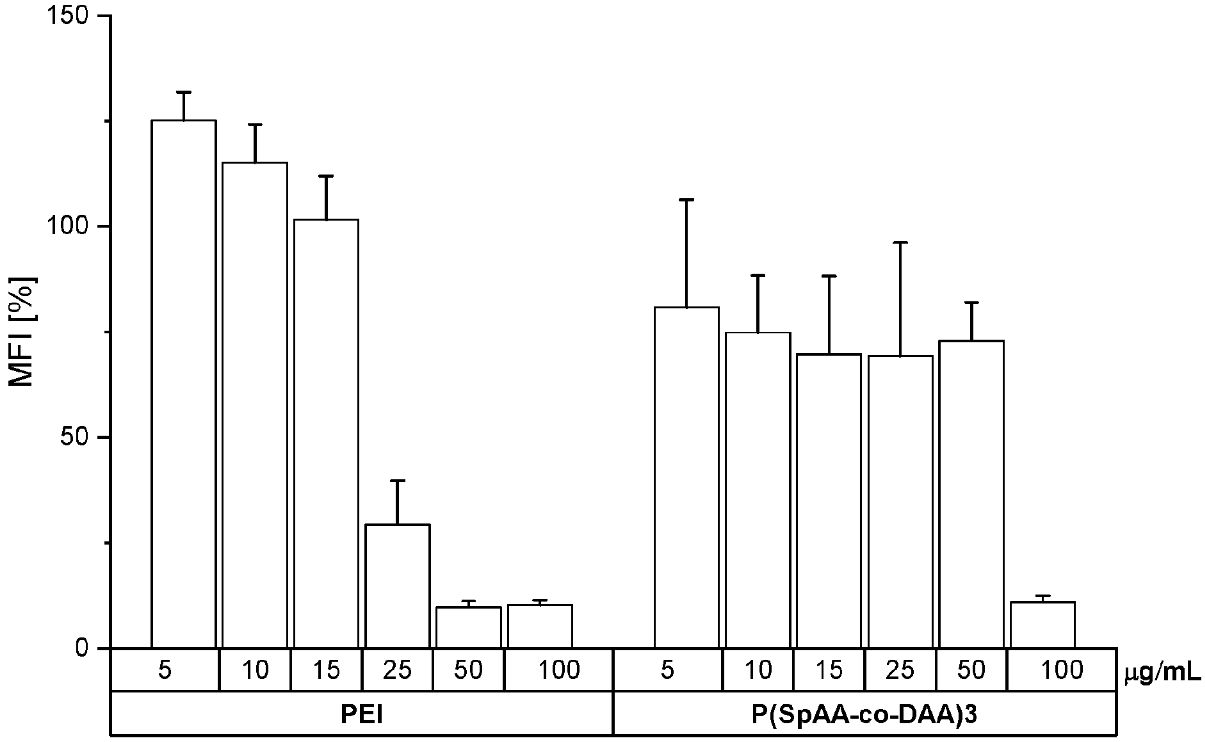


Fig. 12A

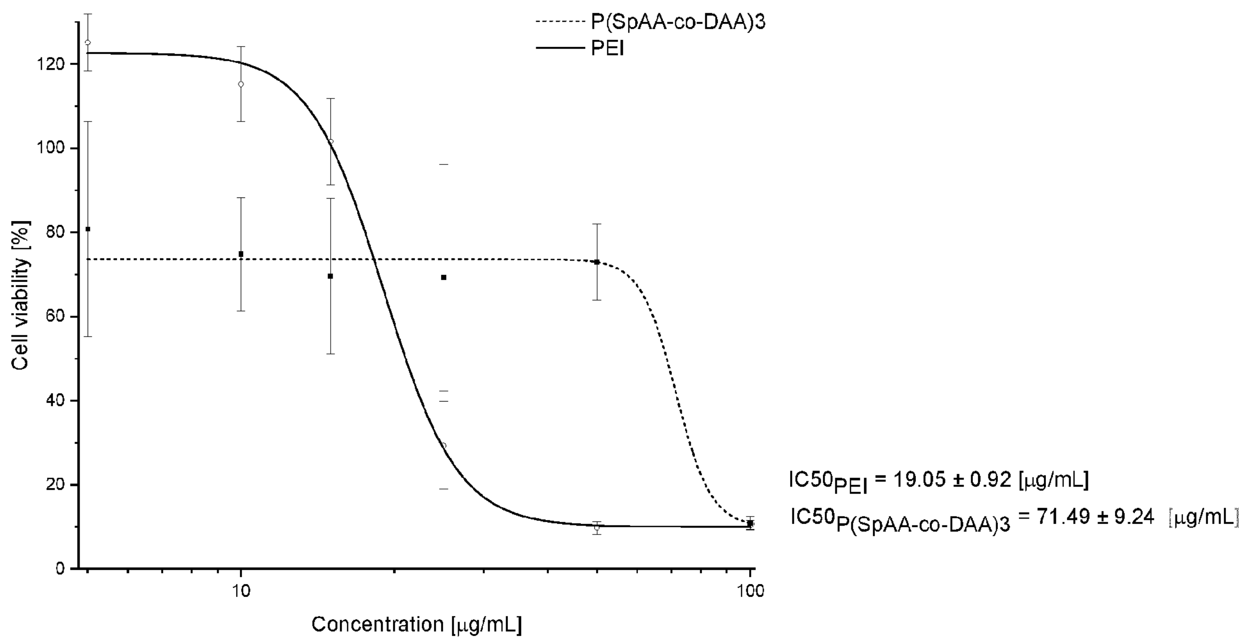


Fig. 12B

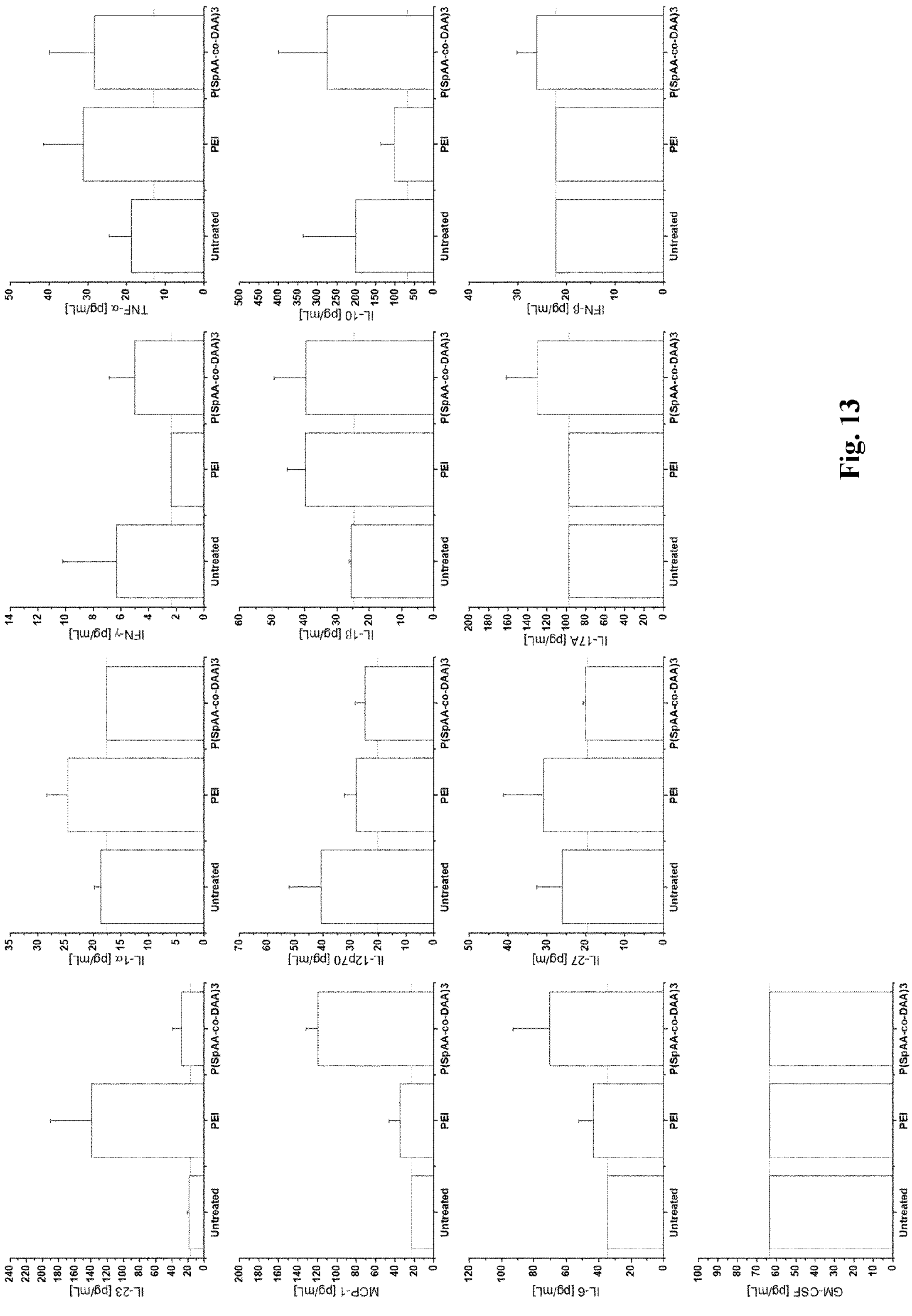
IL-23 [pg/mL]
MCP-1 [pg/mL]
IL-6 [pg/mL]
GM-CSF [pg/mL]
IL-1α [pg/mL]
IL-12p70 [pg/mL]
IL-27 [pg/m]
IFN-γ [pg/mL]
IL-1β [pg/mL]
IL-17A [pg/mL]
TNF-α [pg/mL]
IL-10 [pg/mL]
IFN-β [pg/mL]
Untreated
PEI
P(SpAA-co-DAA)3

Fig. 13

Europäisches Patentamt
European Patent Office
Office européen des brevets

# EUROPEAN SEARCH REPORT

Application Number
EP 22 16 2499

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALFRED POLLAK ET AL: "Enzyme Immobilization by Condensation Copolymerization into Cross-Linked Polyacrylamide Gels<1>", JOURNAL OF THE AMERICAN CHEMICAL SOCIETY, AMERICAN CHEMICAL SOCIETY, vol. 102, no. 20, 1 January 1980 (1980-01-01), pages 6324-6336, XP001252386, ISSN: 0002-7863 * Scheme 1, left column; page 6325 * | 1-6 | INV. C08F2/38 C08F220/54 A61K9/50 C08F4/04 C08F8/30 |
| X | JERE D ET AL: "Akt1 silencing efficiencies in lung cancer cells by sh/si/ssiRNA transfection using a reductable polyspermine carrier", BIOMATERIALS, ELSEVIER, AMSTERDAM, NL, vol. 30, no. 8, 1 March 2009 (2009-03-01), pages 1635-1647, XP025876360, ISSN: 0142-9612, DOI: 10.1016/J.BIOMATERIALS.2008.12.005 [retrieved on 2009-01-19] * 2. Materials and mehtods; page 1636 - page 1639 * | 7-12 | |
| X | WO 2013/138783 A1 (UNIV CALIFORNIA [US]) 19 September 2013 (2013-09-19) * figures 1,2; examples 1,2 * * page 21 - page 23; tables 1-3 * * claims 16-20 * * claims 1,6 * | 7-12 | |
| | | | TECHNICAL FIELDS SEARCHED (IPC) |
| | | | C08F A61K |

The present search report has been drawn up for all claims

1

| Place of search | Date of completion of the search | Examiner |
|---|---|---|
| Munich | 2 August 2022 | Thomas, Dominik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 16 2499

This annex lists the patent family members relating to the patent documents cited in the above-mentioned European search report. The members are as contained in the European Patent Office EDP file on
The European Patent Office is in no way liable for these particulars which are merely given for the purpose of information.

02-08-2022

| Patent document cited in search report | Publication date | Patent family member(s) | Publication date |
| --- | --- | --- | --- |
| WO 2013138783 A1 | 19-09-2013 | AU 2013231839 A1 | 30-10-2014 |
| | | AU 2018201179 A1 | 08-03-2018 |
| | | AU 2020201775 A1 | 26-03-2020 |
| | | CA 2907095 A1 | 19-09-2013 |
| | | JP 6283652 B2 | 21-02-2018 |
| | | JP 6571220 B2 | 04-09-2019 |
| | | JP 2015510896 A | 13-04-2015 |
| | | JP 2018087208 A | 07-06-2018 |
| | | JP 2019189656 A | 31-10-2019 |
| | | US 2015071999 A1 | 12-03-2015 |
| | | US 2018036254 A1 | 08-02-2018 |
| | | US 2020397712 A1 | 24-12-2020 |
| | | WO 2013138783 A1 | 19-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Non-patent literature cited in the description

- Remington's Pharmaceutical Sciences. Mack Publishing Company